# EUROPÄISCHE PATENTSCHRIFT

(11) **EP 2 137 303 B1**
(45) Veröffentlichungstag und Bekanntmachung des Hinweises auf die Patenterteilung: **05.01.2011**
(21) Anmeldenummer: 08734466.9
(22) Anmeldetag: 07.04.2008
(51) Int. Cl.: C12N 9/02, C12P 13/20

(54) **PROTEIN ZUR CHEMOENZYMATISCHEN HERSTELLUNG VON L-THREO-HYDROXYASPARTAT**
PROTEIN FOR CHEMOENZYMATIC PRODUCTION OF L-THREO-HYDROXYASPARTATE
PROTÉINE POUR LA PRODUCTION CHIMIO-ENZYMATIQUE DE L-THREO-HYDROXYASPARTATE

(30) Priorität: 13.04.2007 DE 102007017861
(43) Veröffentlichungstag der Anmeldung: 30.12.2009
(73) Patentinhaber: Philipps-Universität Marburg, 35032 Marburg (DE)
(72) Erfinder: MARAHIEL, Mohamed, A., 35043 Marburg-Cappel (DE); STRIEKER, Matthias, 35037 Marburg (DE); ESSEN, Lars-Oliver, 35043 Marburg (DE)
(74) Vertreter: Buchhold, Jürgen
(86) Internationale Anmeldenummer: PCT/DE2008/000580
(87) Internationale Veröffentlichungsnummer: WO 2008/125080

(56) Entgegenhaltungen:
- STRIEKER M ET AL: "Mechanistic and structural basis of stereospecific C[beta]-hydroxylation in calcium-dependent antibiotic, a daptomycin-type lipopeptide" ACS CHEMICAL BIOLOGY 200703 US, Bd. 2, Nr. 3, März 2007 (2007-03), Seiten 187-196, XP002502363 ISSN: 1554-8929
- STRIEKER MATTHIAS ET AL: "Non-heme hydroxylase engineering for simple enzymatic synthesis of L-threo-hydroxyaspartic acid" CHEMBIOCHEM, Bd. 9, Nr. 3, Januar 2008 (2008-01), Seiten 374-376, XP002502508 ISSN: 1439-4227

## Beschreibung

### Stand der Technik

Im zentralen Nervensystem (ZNS) von Säugetieren nimmt L-Glutamat eine zentrale Rolle ein, indem es als primärer exzitatorischer Neurotransmitter agiert. Dabei nimmt L-Glutamat an verschiedensten neuronalen Kommunikationen teil, bei denen eine große Anzahl an exzitatorischen Aminosäurerezeptoren aktiviert werden, welche für komplexe Signalübertragungen zuständig sind, wie z.B. Gedächtnisausbildung, Lernprozesse, Entwicklung sowie die Immunantwort auf Verletzungen. Allerdings kann L-Glutamat diese Rezeptoren auch überaktivieren und somit zu einem akuten oder chronischen Schaden im ZNS beitragen.

Daher ist eine Regulierung der Menge des exzitatorischen L-Glutamats entscheidend, da ein Mangel an L-Glutamat zu einer verminderten Signalübertragung führt, während ein Überschuss zur Initiierung exzytotoxischer Signalwege führt. Ein Überschuss an L-Glutamat tritt beispielsweise bei Ischämie, Hypoglykämie, Chorea Huntington, Morbus Alzheimer, amyotroper Lateralsklerose, tardiven Dyskinesien, Angststörungen, Depressionen, Schizophrenie, Epilepsie; Astrozytomen und bei manchen Lebererkrankungen auf.

Der Hauptanteil des Glutamattransports im ZNS wird durch hoch affine, Natriumabhängige EAA-Transporter ("excitatory amino acid transporter') vermittelt.

Es konnte gezeigt werden, dass das EAA-Analogon L-*threo*-Hydroxyaspartat die Funktion der Glutamattransporter hemmt (RJ Bridges, MP Kavanaugh, AR Chamberlin: A pharmacological review of competitive inhibitors ans substrates of highaffinity, sodium-dependent glutamate transport in the central nervous system. Curr Pharm Des 1999, 5, 363-379). Diese Inhibitorwirkung weist auch das benzylierte Derivat (L-TBOA) auf (K Shimamoto, B Lebrun, Y Yasuda-Kamatani, M Sakaitani, Y Shigeri, N Yumoto, T Nakajima: DL-threo-beta-benzyloxyaspartate, a potent blocker of excitatory amino acid transporters. Mol Pharmacol 1998, 53, 195-201 und K Shimamoto, Y Shigeri, Y Yasuda-Kamatani, B Lebrun, N Yumoto, T Nakajima: Syntheses of optically pure beta-hydroxyaspartate derivatives as glutamate transporter blockers. Bioorg Med Chem Lett 2000, 10, 2407-2410).
Die Synthese eines racemischen Gemischs von DL-*threo*-Hydroxyaspartat und seiner erythreo-Form wurde erstmals 1921 von Dakin beschrieben. Dieser Syntheseweg beginnt mit Fumarat oder Malat als Ausgangsmaterial. Die ungesättigte Säure wird in Chloromalat überführt und anschließend zu Hydroxyaspartat aminiert (HD Dakin: The synthesis of inactive para- and anti-hydroxyaspartic acids (aminomalic acids). J Biol Chem 1921, 48, 273-291).

Der zweite bekannte Syntheseweg ist im Detail für β-Methyl-β-hydroxyaspartat beschrieben und beinhaltet eine Kondensation vom Aldoltyp zwischen Kupferglycinat und einer Carbonylverbindung (L Benoiton, SM Birnbaum, M Winitz, JP Greenstein: The enzymatic route of beta-methylaspartic acid with acylase II. Arch Biochem Biophys 1959, 81, 434-438). Dieser Syntheseweg führt jedoch zu einem komplexen Diastereomerengemisch.

Die Synthese von optisch reinem L-TBOA, dem wirksamsten Inhibitor der Glutamattransporter, beginnt mit (R)-Garner-Aldehyd, beinhaltet sieben Synthesestufen und ist zeit- und kostenintensiv (K Shimamoto, Y Shigeri, Y Yasuda-Kamatani, B Lebrun, N Yumoto, T Nakajima: Syntheses of optically pure beta-hydroxyaspartate derivatives as glutamate transporter blockers. Bioorg Med Chem Lett 2000, 10, 2407-2410).

Die bislang bekannten Verfahren zur Herstellung von L-*threo*-Hydroxyaspartat sind zeitaufwändig, kostenintensiv und liefern kein enantiomerenreines Produkt. Ein Verfahren zur enantioselektiven Synthese von L-*threo*-Hydroxyaspartat (L-THA) wäre jedoch wünschenswert, da sich aus L-THA sehr einfach L-TBOA herstellen lässt. Die vorliegende Erfindung liefert ein neuartiges Protein, das die chemoenzymatische und enantiomerenreine Herstellung von L-*threo-*Hydroxyaspartat aus L-Aspartat gestattet.

### Aufgabe

Aufgabe der vorliegenden Erfindung ist es, ein Protein bereitzustellen, welches die chemoenzymatische und enantioselektive Synthese von L-*threo*-Hydroxyaspartat aus L-Aspartat katalysiert.

### Lösung der Aufgabe

Diese Aufgabe wird erfindungsgemäß gelöst durch ein Protein umfassend mindestens die Aminosäuren 13-318 von AsnO D241 N, worin
- AsnO D241 N eine einfache Mutante der natürlichen Asparaginoxygenase AsnO darstellt,
- der Aminosäurerest an Position 241 der natürlichen Asparaginoxygenase AsnO von Aspartat (D) zu Asparagin (N) ausgetauscht ist
- unter einfacher Mutante verstanden wird, dass gegenüber dem natürlichen Protein genau eine Aminosäure ausgetauscht ist.

Das natürliche Protein AsnO (Asparaginoxygenase) ist Teil des CDA-Biosynthese-Genclusters in *Streptomyces coelicolor*, wobei CDA "calcium-dependent antibiotic" bedeutet. AsnO ist eine Fe²⁺- und α-Ketoglutarat-abhängige Hydroxylase, welche ausschließlich die Bildung von L-*threo*-Hydroxyasparagin aus L-Asparagin katalysiert. L-*threo*-Hydroxyasparagin dient in vivo als Baustein für nicht ribosomal hergestelltes CDA. Während des Katalysezyklus verbindet diese Klasse von Enzymen den oxidativen Abbau von α-Ketoglutarat zu Succinat und CO₂ mit der Hydroxylierung des Substrates (L-Asparagin).

Im Wildtyp des AsnO bindet dabei die Seitenkette des Aminosäurerestes Asp-241 an die NH₂-Gruppe der Carboxamidgruppe von L-Asn.

Überraschend wurde gefunden, dass eine gerichtete Mutagenese von Asp-241 zu Asn-241 (D241 N) zu einer Bindungsstelle für die Carboxylgruppe einer Aspartatseitenkette führt. Durch diese gerichtete Mutagenese ändert sich die Substratspezifität von Asparagin zu Aspartat, und das mutierte Protein überführt Aspartat in Gegenwart von Fe²⁺- und α-Ketoglutarat (α-KG) chemoenzymatisch in L-*threo-*Hydroxyaspartat. Die Umsetzung erfolgt quantitativ und enantioselektiv nach folgendem Schema:

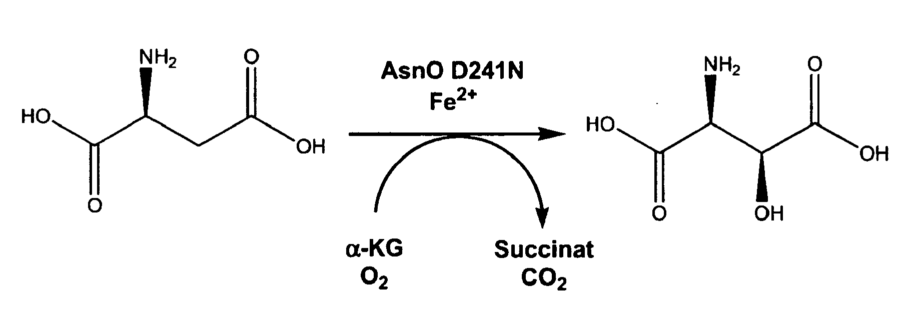

Das auf diese Weise mutierte Protein wird nachfolgend als AsnO D241 N bezeichnet. Erfindungsgemäß wird AsnO D241 N als "einfache Mutante" des Wildtyps AsnO bezeichnet. Hierin bedeutet "einfache Mutante", dass in AsnO D241N genau eine Aminosäure (hier: Aminosäure 241) im Vergleich zum Wildtyp verändert ist.

Überraschend wurde gefunden, dass ein Protein, welches mindestens die Aminosäuresequenz des AsnO D241 N vom ersten bis zum letzten Sekundärstrukturelement enthält, L-Aspartat in das korrespondierende L-*threo*-Hydroxyaspartat überführt. Dabei bedeutet "vom ersten bis zum letzten Strukturelement", dass mindestens die Sequenz von Aminosäure D13 (Aspartat) bis M318 (Methionin) von AsnO D241 N vorliegen muss.
In einer besonders bevorzugten Ausführungsform umfasst das Protein, welches die Umwandlung von L-Asp zu L-*threo*-Hydroxyaspartat katalysiert, die Aminosäuren A2 (Alanin) bis A333 (Alanin) von AsnO D241 N.

Erfindungsgemäß wird ein Protein umfassend mindestens die Aminosäuren 13-318 von AsnO D241 N hergestellt, indem zunächst eine gerichtete Mutagenese des Gens des AsnO-Wildtyps durchgeführt wird; anschließend erfolgt die Klonierung dieses Gens in einen Vektor und zuletzt wird ein Wirtsorganismus mit dem Expressionvektor transformiert und das rekombinante Protein exprimiert. Erfindungsgemäß wird bei der gerichteten Mutagenese das Codon GAC (Basen 721-723), welches im Wildtyp für Asp-241 codiert, gegen ein Codon ausgetauscht, das für Asn codiert. Dem Fachmann ist bekannt, dass die Codons AAC und AAT für Asparagin codieren. Erfindungsgemäß kann die gerichtete Mutagenese im Austausch von GAC gegen AAC oder von GAC gegen AAT bestehen. In einer bevorzugten Ausführungsform wird bei der gerichteten Mutagenese GAC gegen AAC ausgetauscht. Dem Fachmann ist bekannt, dass er die gerichtete Mutagenese durch die Auswahl geeigneter Oligonukleotidprimer mit entsprechenden Mutagenesestellen durchführen kann. Er kann dieses Wissen anwenden, ohne den Schutzbereich der Patentansprüche zu verlassen.

In Analogie zur Definition des Begriffs "einfache Mutante" auf Proteinebene bedeutet "einfache Mutante" auf Nukleinsäureebene, dass genau ein Codon (hier: Basen 721-723) im Vergleich zum Wildtyp verändert ist.

Dem Fachmann ist bekannt, wie er Oligonukleotide in Vektoren klonieren kann. Ein geeigneter Vektor ist beispielsweise pQTEV.

Als Wirtsorganismus, der mit dem Expressionvektor transformiert werden und dann das rekombinante Protein exprimieren kann, eignen sich dem Fachmann bekannte Bakterienstämme, beispielsweise *Eschericia coli.*

Das erfindungsgemäße Protein umfassend mindestens die Aminosäuren 13-318 von AsnO D241 N wird hergestellt durch ein Verfahren umfassend folgende Schritte:
a) Herstellung eines Oligonukleotids (Gen), das mindestens die Basen 37-954 der DNA-Sequenz von AsnO umfasst,
b) gerichtete Mutagenese dieses Gens, wobei das Aspartatcodon der Basen 721-723 von AsnO gegen ein Asparagincodon ausgetauscht wird,
c) Klonierung dieses Gens in einen Expressionsvektor,
d) Transformation eines Wirtsorganismus mit dem Expressionsvektor und Expression des rekombinanten Proteins.

In einer bevorzugten Ausführungsform wird das Expressionsplasmid in Form eines His-Fusionsplasmids hergestellt, welches zur Expression eines erfindungsgemäßen Proteins mit einem N-terminalen His-Tag führt. Bevorzugt handelt es sich dabei um Tags mit 5 bis 8 aufeinander folgenden Histidinresten. Dem Fachmann ist bekannt, dass sich derartige His-Tag-Fusionsproteine vorteilhaft mittels Affinitätschromatographie über eine Ni-Chelatsäule reinigen lässt.

Das erfindungsgemäße Protein kann zur Herstellung von L-*threo*-Hydroxyaspartat verwendet werden. Hierfür wird L-Aspartat in Gegenwart des erfindungsgemäßen Proteins und dem Cofaktor Fe²⁺- sowie mit dem Cosubstrat α-Ketoglutarat inkubiert. Dabei wird eine nicht aktivierte β-Methylengruppe (hier: die β-CH₂-Gruppe von Aspartat) enzymatisch hydroxyliert. Diese enzymatische Hydroxylierung ist besonders vorteilhaft, da nicht aktivierte β-CH₂-Gruppen auf klassisch chemischem Wege im Allgemeinen nur schwer zugänglich sind. "Rein chemische", nicht enzymkatalysierte Reaktionen an β-CH₂-Gruppen von Aminosäuren führen außerdem in aller Regel zu Enantiomeren- bzw. Diastereomerengemischen. Die chemoenzymatische Herstellung von L-*threo*-Hydroxyaspartat mit Hilfe des erfindungsgemäßen Proteins verläuft dagegen enantioselektiv und substratspezifisch, da AsnO D241 N ausschließlich L-Aspartat hydroxyliert und ausschließlich L-*threo-*Hydroxyaspartat gebildet wird.

### Ausführungsbeispiele

### Ausführungsbeispiel 1

### Herstellung des Expressionsplasmids und gerichtete Mutagenese

Es wird eine gerichtete Mutagenese des AsnO-Wildtyps durchgeführt, wobei das Codon für Asp-241 des Wildtyps zu Asn-241 verändert wird. Das verwendete AsnO ist Bestandteil des CDA-Biosynthese-Genclusters in *Streptomyces coelicolor,* wobei CDA "calcium-dependent antibiotic" bedeutet. Die DNA-Sequenz des AsnO-Wildtyps zeigt SEQ ID No: 1.

Die rekombinanten Genfragmente werden mittels Polymerasekettenreaktion aus chromosomaler DNA von *Streptomyces coelicolor* A3(2) (DSM 40783) unter Verwendung der Phusion-Polymerase (Finnzymes) amplifiziert. Gemäß den Herstellerangaben für Templat-DNA mit hohem GC-Gehalt (*S. coelicolor,* 74 %) wird die dNTP-Konzentration auf 20 mM erhöht.

Für die Durchführung der gerichteten Mutagenese wird das QuickChange II Site-directed Mutagenesis Kit (Stratagene) gemäß Herstellerangaben verwendet. Es werden die synthetischen Oligonukleotidprimer (Operon)
5'-CCCCGACCTGCGGGTGAACCTGGCGGCCACCGAGC-3' (SEQ ID No: 5) und 5'-GCTCGGTGGCCGCCAGGTTCAC CCGCAGGTCGGGG-3' (SEQ ID No: 6) verwendet, die Mutagenesestelle ist unterstrichen.

Die Identität des so hergestellten mutierten Plasmids wird mittels DNA-Dideoxysequenzierung bestätigt.

SEQ ID No: 3 zeigt die DNA-Sequenz von AsnO D241 N; SEQ ID No: 11 zeigt die DNA-Sequenz der korrespondierenden His₇-Fusion (1.074 kb).

### Ausführungsbeispiel 2

### Herstellung des rekombinanten Enzyms

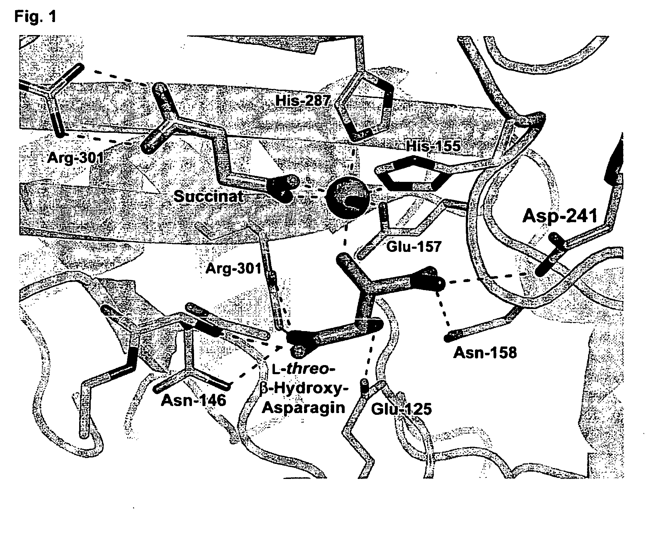

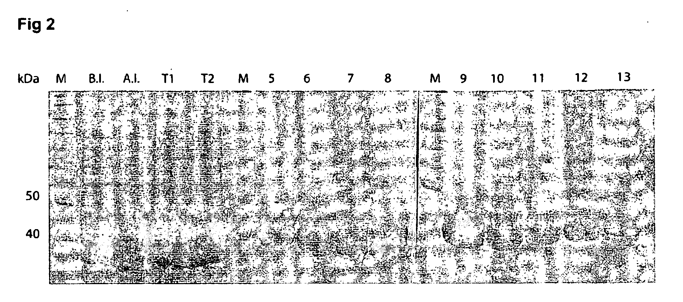

Das mutierte AsnO-Gen wird in die Bam HI- und Not I-Schnittstellen des pQTEV-Vektors (SEQ ID No: 17, GenBank Accession Number AY_243506) kloniert. Dieses pQE30-basierte (Qiagen) Klonierungsprodukt wird für die Transformation von *E. coli* BL21(DE3) (Novagen) verwendet. Die transformierten Zellen werden bei 37 °C bis zu einer optischen Dichte von 0.5 (λ= 600 nm) kultiviert, mit 1 mM Isopropyl-β-D-thiogalactopyranosid induziert und nach weiteren 3 h bei 30 °C geerntet. Die rekombinanten Proteine werden mittels Ni-NTA-Affinitätschromatographie (Amersham Pharmacia Biotech) gereinigt. Ein 12% SDS-PAGE-Gel zur Kontrolle der Reinigung ist in Fig. 2 gezeigt.

Die Bahnen mit gereinigtem Protein werden aus dem Gel ausgeschnitten, vereinigt und einem Pufferaustausch in 25 mM HEPES, 50 mM NaCl, pH 7,0 unter Verwendung von HiTrap-Entsalzungssäulen (Amersham Pharmacia Biotech (siehe Paper ACS Chemical Biology und Papermanuscript) unterworfen. Die Konzentration des gereinigten Proteins wird spektrophotometrisch unter Verwendung berechneter Extinktionskoeffizienten bei 280 nm bestimmt. Nach Schockfrosten in flüssigem Stickstoff wird das gereinigte Protein bis zur weiteren Verwendung bei -80 °C gelagert.

SEQ ID No: 2 zeigt die Aminosäuresequenz des AsnO-Wildtyps.

SEQ ID No: 12 zeigt die Aminosäuresequenz des AsnO D241 N His₇-Tags;

SEQ ID No: 10 zeigt die Aminosäuresequenz von AsnO D241 N ohne Klonierungsartefakte, diese sind der His₇-Tag, eine kurze Verbindungsregion und die "Tobacco Etch Virus Protease" Erkennungsstelle. Bei Aminosäure 1 des AsnO-Wildtyps handelt es sich um Methionin. Durch Verwendung des pQTEV-Vektors wird das Methionin-Codon des AsnO-Wildtyps zu einem Serin-Codon verändert, so dass bei SEQ ID No: 8 und SEQ ID No. 10 die erste Aminosäure des AsnO D241 N Serin und nicht Methionin ist.

### Ausführungsbeispiel 3

### Aktivität der AsnO D241N-Mutante

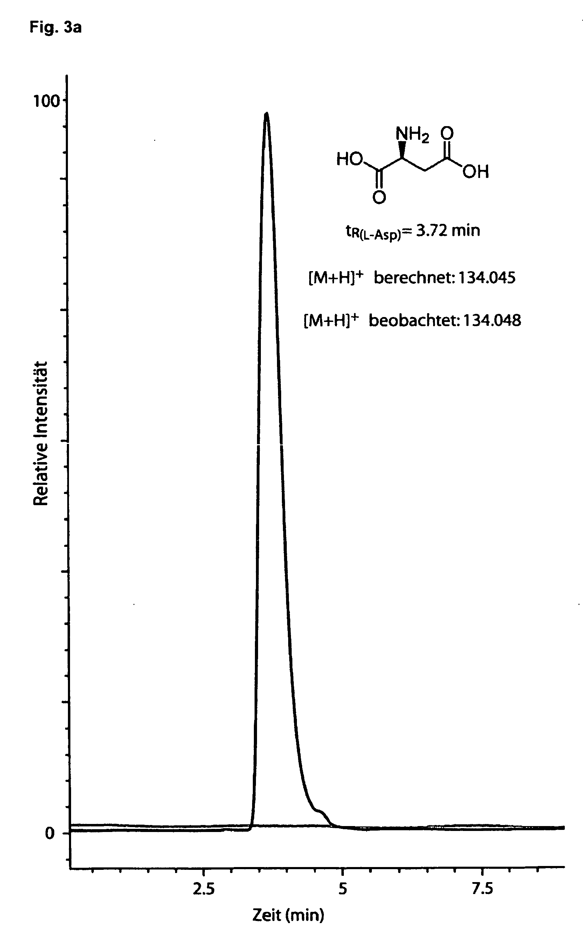

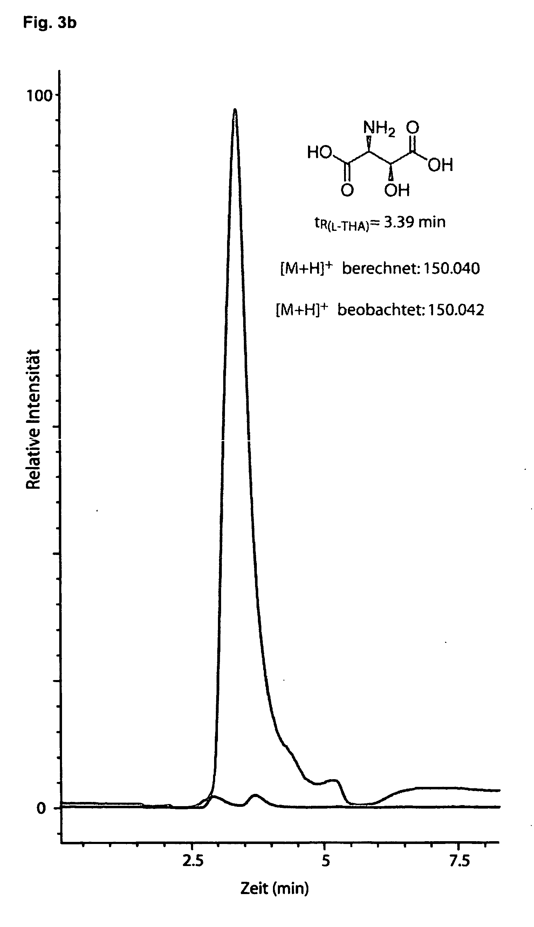

Um die Aktvität der AsnO D241 N-Mutante zu evaluieren, wird das gereinigte Enzym AsnO D241N (40 µM) mit 1,5 mM L-Asp, 1,0 mM (NH₄)₂Fe(SO₄)₂ als Quelle für den Eisen-Cofaktor und 1,0 mM α-Ketoglutarat (Cosubstrat) bei verschiedenen Temperaturen zwischen 16 °C und 37 °C für 16 h inku biert. Die Reaktion wird mittels HPLC-MS verfolgt, indem die Massen für L-Asp ([M+H]⁺ = 134.5 Da) und seine hydroxylierte Form ([M+H]⁺ = 150.04 Da) gescannt werden. Die Inkubierung von L-Aspartat mit AsnO D241 N führt zu dessen quantitativer Umwandlung in L-*threo*-3-Hydroxyaspartat bei 16 °C (Fig. 3a). In der Kont rollreaktion, bei der kein Enzym zugegeben wird, ist lediglich L-Asp nachweisbar (Fig. 3b). Kommerziell erhältliches L-Asp (Bachem) sowie L-*threo*-3-Hydroxyaspartat (Tocris Bioscience) werden als Standards für den Vergleich der Retentionszeiten bei der HPLC verwendet.

### Ausführungsbeispiel 4

### Evaluierung der kinetischen Parameter

Für die Evaluierung der kinetischen Parameter verschiedenen Konzentrationen des Substrates L-Asp (50 µM bis 2 mM) wie unter Ausführungsbeispiel 3 beschrieben mit AsnO D241N (40 µM), 1,0 mM (NH₄)₂Fe(SO₄)₂ als Quelle des Eisen-Cofaktors und 1,0 mM α-Ketoglutarat (Cosubstrat) inkubiert und der Enzymassay zu verschiedenen Zeitpunkten durch Zugabe von Nonafluorpentansäure gestoppt. Nonafluorpentansäure fungiert außerdem als Ionenpaarbildungsreargenz bei HPLC-Analysen.

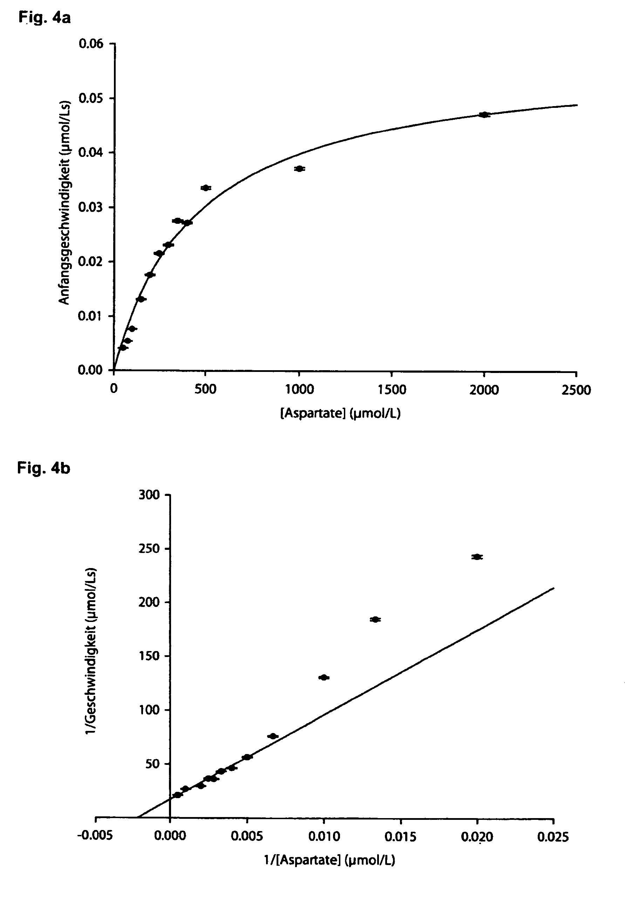

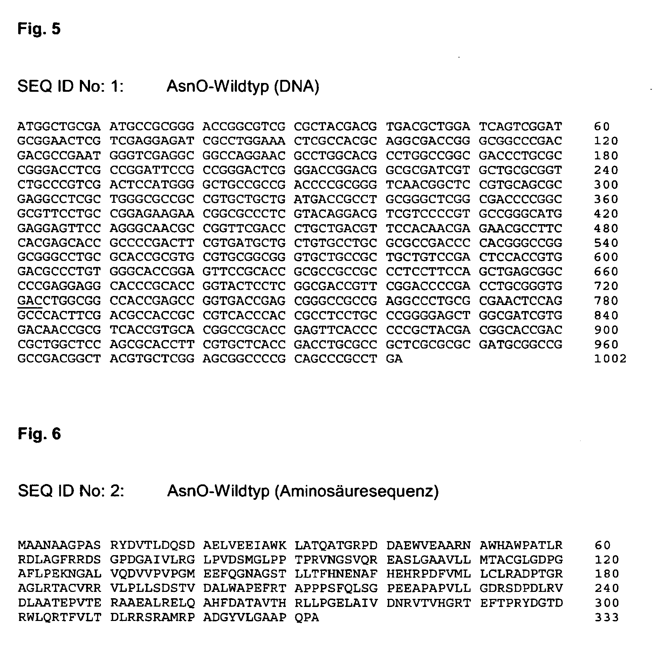

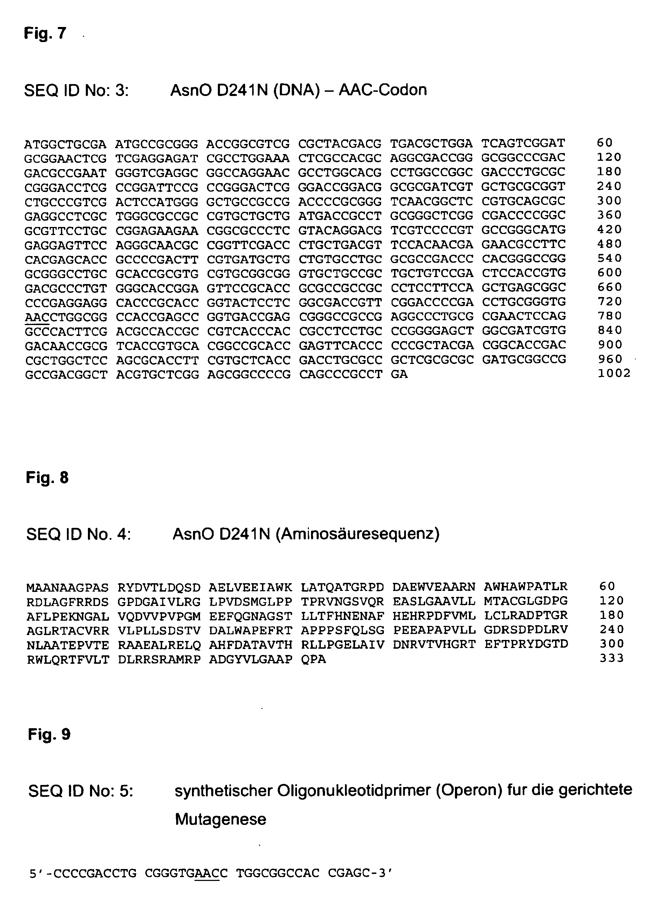

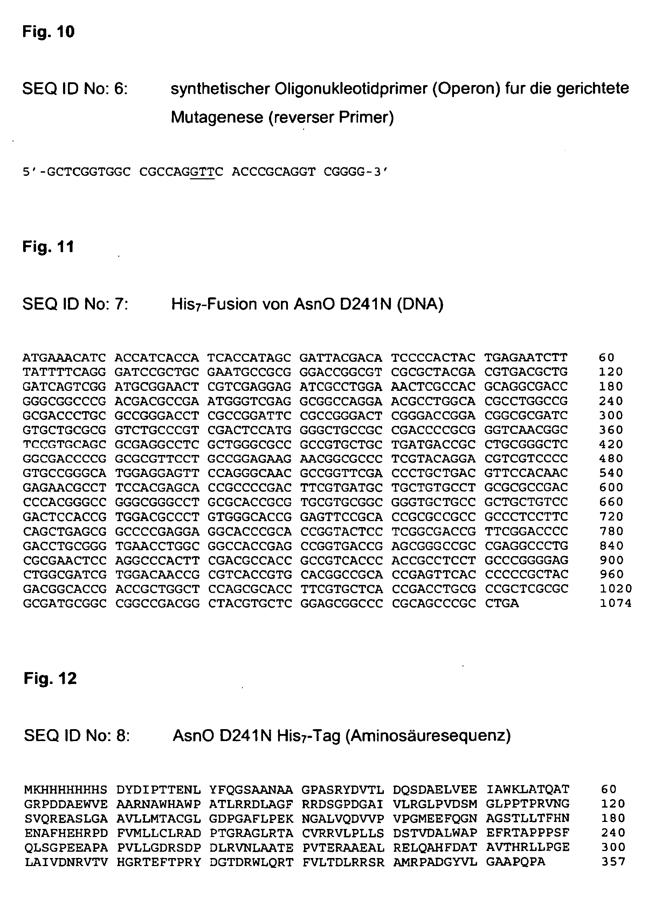

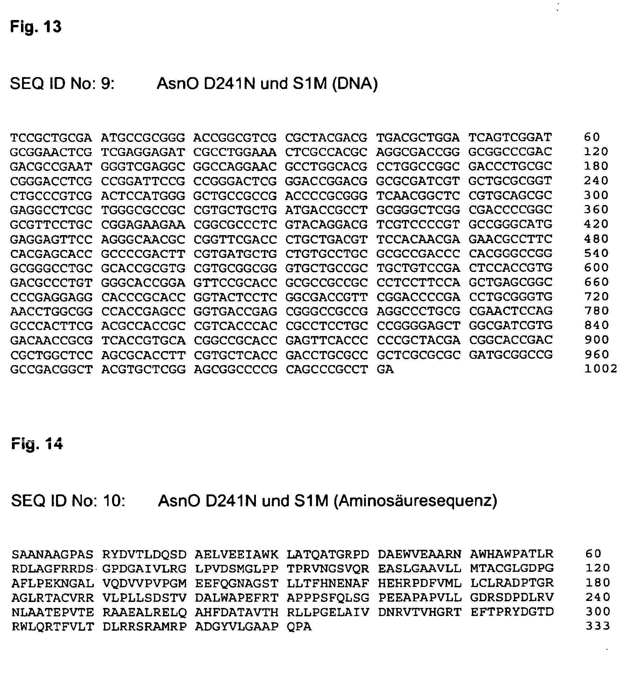

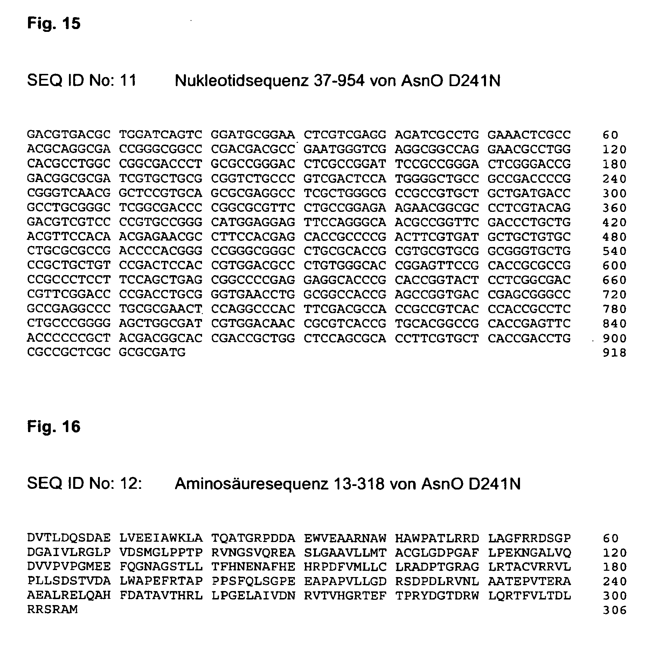

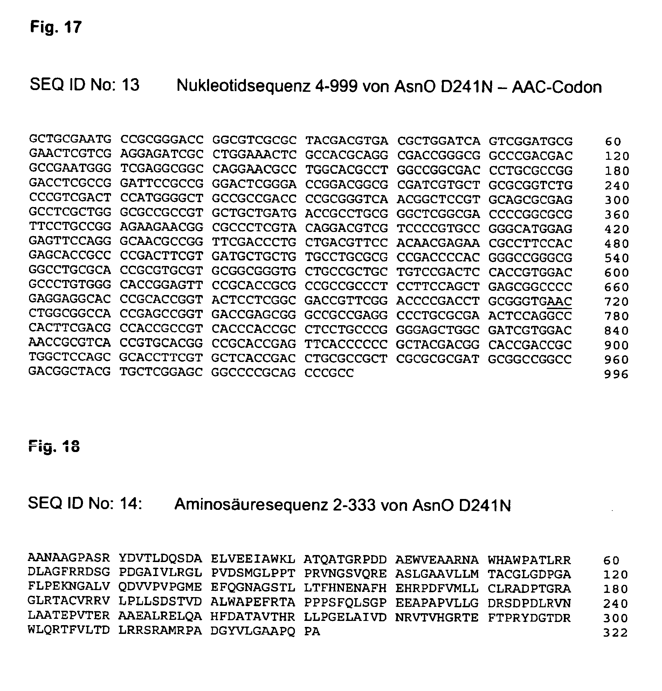

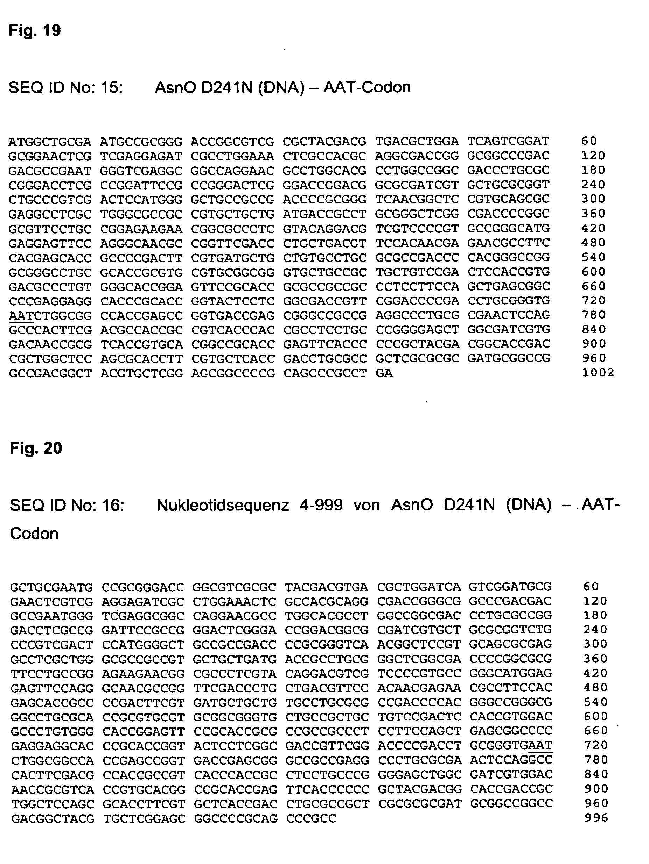

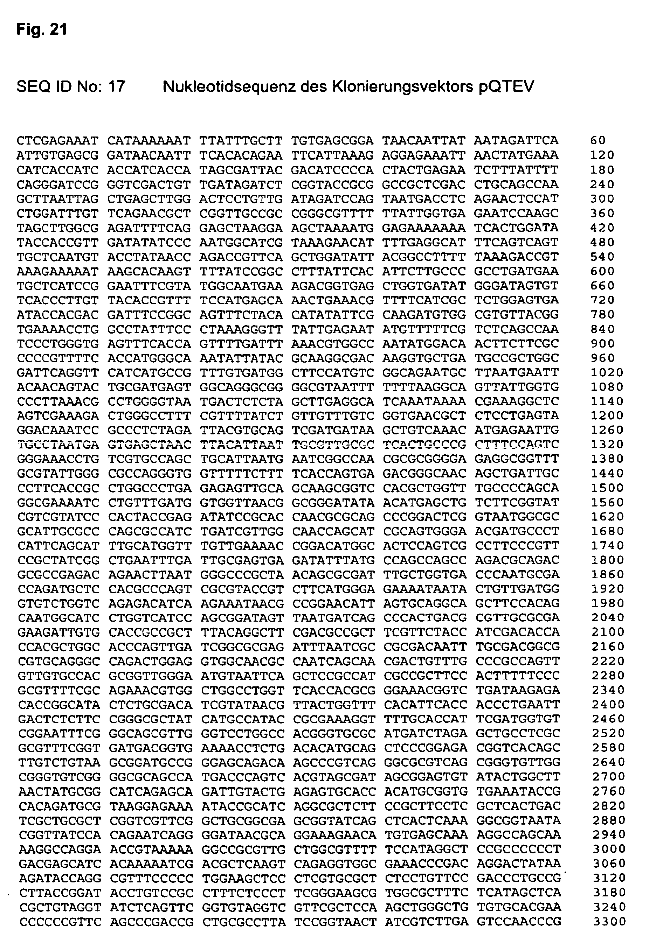

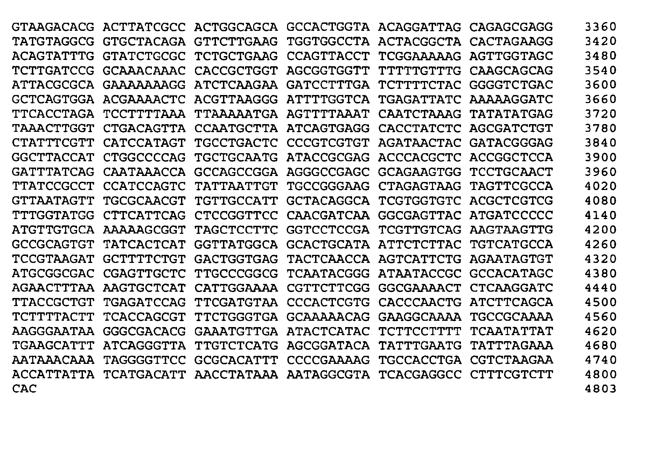

Die graphische Auftragung der Anfangsgeschwindigkeiten gegen die Substratkonzentrationen zeigt, dass es sich um eine Michaelis-Menten-Kinetik handelt (Fig. 4a).

Die kinetischen Parameter von AsnO D241 N werden anschließend über eine Lineweaver-Burk-Gleichung ermittelt (Fig. 4b).

Für AsnO D241 N ergibt sich K_{M} = 0.457 ± 0.031 mM mit k_{cat} = 1.0 ± 0.1 min⁻¹ für L-Asp. Die katalytische Effizienz beträgt k_{cat}/K_{M}= 2.2 ± 0.4 min⁻¹*mM⁻¹. Im Vergleich dazu weist der Wildtyp AsnO einen nahezu identischen K_{M}-Wert von 0.478 ± 0.067 mM für L-Asn auf, besitzt aber einen 300-fach höheren k_{cat} (298 ± 19 min⁻¹).

**Tab. 1: Substratspezifität von AsnO D241 N**

| Aminosäure | [M+H]⁺ berechnet | [M+H]⁺ Hydroxylierungs-produkt | [M+H]⁺ gefunden | Hydroxylierung |
|---|---|---|---|---|
| L-Asp | 134.0 | 150.0 | 150.0 | ja |
| D-Asp | 134.0 | 150.0 | 134.1 | nein |
| L-Asn | 133.1 | 149.1 | 133.0 | nein |
| L-Gln | 147.1 | 163.1 | 147.2 | nein |
| L-Glu | 148.1 | 164.1 | 148.2 | nein |
| L-Ile | 132.1 | 148.1 | 132.2 | nein |
| L-Phe | 166.1 | 182.1 | 166.1 | nein |
| L-Trp | 205.1 | 221.1 | 205.1 | nein |
| L-Val | 118.1 | 134.1 | 118.0 | nein |

### Ausführungsbeispiel 5

### Substratspezifität des mutierten Proteins AsnO D241 N

Die Spezifität von AsnO D241 N für die Umwandlung von L-Asp in L-*threo*-3-Hydroxyaspartat wird untersucht, indem dieses mutierte Protein während 16 h bei 16 °C mit den in Tab. 1 aufgeführten Aminosäuren (1 ,5 mM), dem Cofaktor (NH₄)₂Fe(SO₄)₂ (1 mM), dem Cosubstrat α-Ketoglutarat (4 mM) und 20 µg Catalase in 250 bis 1 mL eines 50 mM HEPES-Puffers (pH 7,5) inkubiert wird. Die Catalase wird zugefügt, um reaktive Sauerstoffspezies abzufangen und somit die Autoxidation des Enzyms zu verhindern. Kontrollen werden in Abwesenheit von AsnO D241 N durchgeführt.

Die Reaktion wird durch Zugabe von 50 - 200 µL einer 4%-igen Lösung (V/V) von Nonafluorpentansäure gestoppt und danach die Hydroxylierung des Aminosäuresubstrates zur jeweils korrespondierenden Hydroxyaminosäure mittels reversed phase HPLC/MS untersucht. Säule: Hypercarb (Thermo Electron Corporation, Porendurchmesser 250 Å, Teilchengröße 5 µM, 100 % Kohlenstoff). Mobile Phasen: A= 20 mM wässrige Nonafluorpentansäure, B= Acetonitril. Gradient: 0 - 10 % B in 12 min, Flussrate 0,2 mL / min bei 17,5 °C.

Das Reaktionsprodukt der Inkubation der jeweiligen Aminosäure mit AsnO D241 N wird mittels höchstauflösender MS an einem API Qstar Pulsar I durchgeführt (Applied Biosystems).

### Abbildungslegenden

**Fig 1**
Kristallstruktur von AsnO mit gebundenem L-*threo*-β-Hydroxyasparagin und Succinat (PDB Accession Code: 2OG7). Die Mutante Asp-241 zu Asn-241 stabilisiert Asp innerhalb der Bindungsstelle und ändert damit die Substratspezifität von L-Asn zu L-Asp.
**Fig. 2**
Fig. 2 zeigt ein 12 % SDS-PAGE der Reinigung von AsnO D241 N mittels Ni-NTA-Affinitätschromatographie.

| | |
|---|---|
| M | Proteinmarker (Fermentas, *PageRuler*) |
| B.I. | vor Induktion |
| A.I. | nach Induktion |
| T1 | Erste Durchflussfraktion des Rohlysats |
| T2 | Letzte Durchflussfraktion des Rohlysats |
| Bahnen 5-13: | Elutionsfraktionen der Ni-NTA-Affinitätschromatographie. Elution erfolgte durch kompetitive Verdrändung des His₇-Tags durch eine Imidazollösung (250 mM, gepuffert mit 50 mM HEPES und 100 mM NaCl, pH=7.5). |

**Fig. 3**
Quantitative Umwandlung von L-Asp in L-*threo*-3-Hydroxyaspartat; Nachweis mittels HPLC-MS.
Säule: Hypercarb (Thermo Electron Corporation, Porendurchmesser 250 Å, Teilchengröße 5 µM, 100 % Kohlenstoff). Mobile Phasen: A= 20 mM wässrige Nonafluorpentansäure, B= Acetonitril. Gradient: 0 - 10 % B in 12 min, Flussrate 0,2 mL / min bei 17,5 °C.
- Fig. 3a:: Negativkontrolle, keine Zugabe von Enzym (= AsnO D241 N). Es wird lediglich L-Asp nachgewiesen.
Retentionszeit: 3.72 min;
M_{ber} = 134.045, M_{gef} = 134.048.
- Fig. 3b:: Inkubation von L-Asp mit AsnO D241 N. Nach 16 h Inkubation bei 16 °C wird ausschließlich L-*threo*-3-Hydroxyaspartat nachgewiesen.
Retentionszeit 3.39 min;
M_{ber} = 150.040, M_{gef} = 150.042.
Der Nachweis erfolgt über hochaufgelöste MS.
**Fig. 4**
4a: Michaelis-Menten-Diagramm für die Umwandlung von L-Asp in L-*threo*-3-Hydroxyaspartat durch AsnO D241 N.
4b: Lineweaver-Burke-Diagramm für die Umwandlung von L-Asp in L-*threo*-3-Hydroxyaspartat durch AsnO D241 N.

**Fig. 5**
- SEQ ID No: 1: AsnO-Wildtyp (DNA)
GenBank Accession Number des AsnO-Wildtyps (DNA): NC_003888
Gen: Komplement (3587687...3588688)
Locus_tag="SC03236"
Synonym: "SCE29.05c"
**Fig. 6**
- SEQ ID No: 2: AsnO-Wildtyp (Aminosäuresequenz)
GenBank Accession Number des AsnO-Wildtyps (Protein): NP_627448
**Fig. 7**
- SEQ ID No: 3: AsnO D241 N (DNA) - AAC-Codon
Basen 721-723: AAC statt GAC im Wildtyp des AsnO.
**Fig. 8**
- SEQ ID No. 4: AsnO D241 N (Aminosäuresequenz)
Mutatante der natürlichen Asparaginoxygenase AsnO; Asp-241 wurde gegen Asn-241 ausgetauscht
**Fig. 9**
- SEQ ID No: 5: synthetischer Oligonukleotidprimer (Operon) für die gerichtete Mutagenese des AsnO-Wildtyps.
Austausch von GAC (Wildtyp) gegen AAC (AsnO D241N).

**Fig. 10**
- SEQ ID No: 6: synthetischer Oligonukleotidprimer (Operon) für die gerichtete Mutagenese des AsnO-Wildtyps (reverser Primer)
Reverser Primer: Austausch von GTC (Wildtyp) gegen GTT (AsnO D241 N)

**Fig. 11**
- SEQ ID No: 7: His₇-Fusion von AsnO D241 N (DNA)
(His7-Fusionsinsert für die Expression von AsnO D241N)

**Fig. 12**
- SEQ ID No: 8: AsnO D241 N His₇-Tag (Aminosäuresequenz)
Sequenz des exprimierten Proteins nach Aufreinigung über Ni-NTA-Affinitätschromatographie

**Fig. 13**
- SEQ ID No: 9: AsnO D241 N und M1 S (DNA)
Basensequenz von AsnO D241 N ohne Klonierungsartefakte; diese sind der Hier Tag, eine kurze Verbindungsregion und die "Tobacco Etch Virus Protease" Erkennungsstelle.
**Fig. 14**
- SEQ ID No: 10: AsnO D241 N und M1S (Aminosäuresequenz)
Aminosäuresequenz von AsnO D241 N ohne Klonierungsartefakte; diese sind der His₇-Tag, eine kurze Verbindungsregion und die "Tobacco Etch Virus Protease" Erkennungsstelle.
**Fig. 15**
- SEQ ID No: 11: Nukleotidsequenz 37-954 von AsnO D241 N

**Fig.16**
- SEQ ID No: 12: Aminosäuresequenz 13-318 von AsnO D241 N

**Fig. 17**
- SEQ ID No: 13: Nukleotidsequenz 4-999 von AsnO D241 N

**Fig. 18**
- SEQ ID No: 14: Aminosäuresequenz 2-333 von AsnO D241 N

**Fig. 19**
- SEQ ID No: 15: AsnO D241 N (DNA)-AAT-Codon
Basen 721-723: AAT statt GAC im Wildtyp des AsnO.
**Fig. 20**
- SEQ ID No: 16: Nukleotidsequenz 4-999 von AsnO D241N - AAT-Codon

**Fig. 21**
- SEQ ID No: 17: Nukleotidsequenz des Klonierungsvektors pQTEV
GenBank Accession Number: AY_243506

## Patentansprüche

1. Protein umfassend mindestens die Aminosäuren 13-318 von AsnO D241N, worin
- AsnO D241 N eine einfache Mutante der natürlichen Asparaginoxygenase AsnO darstellt,
- der Aminosäurerest an Position 241 der natürlichen Asparaginoxygenase AsnO von Aspartat (D) zu Asparagin (N) ausgetauscht ist
- unter einfacher Mutante verstanden wird, dass gegenüber dem natürlichen Protein genau eine Aminosäure ausgetauscht ist.

2. Protein gemäß Anspruch 1, umfassend eine Aminosäuresequenz gemäß SEQ ID No: 12.

3. Protein gemäß Anspruch 1, umfassend eine Aminosäuresequenz gemäß SEQ ID No: 14.

4. Verfahren zur Herstellung eines Proteins gemäß Anspruch 1, umfassend folgende Schritte:
e) Herstellung eines Oligonukleotids (Gen), das mindestens die Basen 37-954 der DNA-Sequenz von AsnO umfasst,
f) Gerichtete Mutagenese dieses Gens, wobei das Aspartatcodon der Basen 721-723 von AsnO gegen ein Asparagincodon ausgetauscht wird,
g) Klonierung dieses Gens in einen Expressionsvektor,
h) Transformation eines Wirtsorganismus mit dem Expressionsvektor und Expression des rekombinanten Proteins.

5. Verfahren gemäß Anspruch 4, **dadurch gekennzeichnet, dass** bei der gerichteten Mutagenese das Aspartatcodon der Basen 721-723 von AsnO gegen das Asparagincodon AAC ausgetauscht wird.

6. Verfahren gemäß Anspruch 4, **dadurch gekennzeichnet, dass** bei der gerichteten Mutagenese das Aspartatcodon der Basen 721-723 von AsnO gegen das Asparagincodon AAT ausgetauscht wird.

7. Verfahren gemäß einem der Ansprüche 4 bis 6, **dadurch gekennzeichnet, dass** das Expressionsplasmid ein His-Fusionsplasmid ist.

8. Verfahren gemäß einem der Ansprüche 4 bis 7, **dadurch gekennzeichnet, dass** als Vektor pQTEV verwendet wird.

9. Verfahren gemäß einem der Ansprüche 4 bis 8, **dadurch gekennzeichnet, dass** als Wirtsorganismus *Eschericia coli* verwendet wird.

10. Protein, erhältlich durch ein Verfahren gemäß einem der Ansprüche 4 bis 9.

11. Verwendung eines Proteins gemäß Anspruch 1 zur chemoenzymatischen und enantioselektiven Herstellung von L-*threo*-Hydroxyaspartat.

## Claims

1. Protein comprising at least the amino acids 13-318 of AsnO D241 N, wherein
- AsnO D241 N represents a simple mutant of the natural asparagine oxygenase AsnO,
- the amino acid residue at position 241 of the asparagine oxygenase AsnO is exchanged from aspartate (D) to asparagine (N)
- under simple mutant, it is understood that compared to the natural protein exactly one amino acid is exchanged.

2. Protein according to claim 1, comprising an amino acid sequence according SEQ ID No: 12.

3. Protein according to claim 1, comprising an amino acid sequence according SEQ ID No: 14.

4. Method for the production of a protein according to claim 1, comprising the following steps:
a) production of an oligonucleotide (gene) which comprises at least the bases 37-954 of the DNA sequence of AsnO,
b) directed mutagenesis of this gene, wherein the aspartate codon of the bases 721-723 of AsnO is replaced with an asparagine codon,
c) cloning of this gene into an expression vector,
d) transformation of a host organism with the expression vector and expression of the recombinant protein.

5. Method according to claim 4, wherein the aspartate codon of the bases 721-723 of AsnO is exchanged with the asparagine codon AAC during the directed mutagenesis.

6. Method according to claim 4, wherein the aspartate codon of the bases 721-723 of AsnO is exchanged with the asparagine codon AAT during the directed mutagenesis.

7. Method according to one of the claims 4 to 6, wherein the expression plasmid is a His-fusion plasmid.

8. Method according to one of the claims 4 to 7, wherein pQTEV is used as the vector.

9. Method according to one of the claims 4 to 8, wherein *Eschericia coli* is used as the host organism.

10. Protein obtainable by a method according to one of the claims 4 to 9.

11. Use of a protein according to claim 1 for the chemoenzymatic and enantioselective production of L-*threo*-hydroxyaspartate.

## Revendications

1. Protéine comprenant au moins les acides aminés 13-318 de l'asparaginoxygénase naturelle AsnO D241 N, où
- AsnO D 241 N représente un simple mutant de l'asparaginoxygénase naturelle AsnO,
- le résidu d'acide aminé en position 241, aspartate (D), de l'asparaginoxygénase naturelle AsnO est remplacé par asparagine (N),
- on entend par simple mutant que par rapport à la protéine naturelle exactement un acide aminé est remplacé.

2. Protéine selon la revendication 1, comprenant une séquence d'acide aminé selon SEQ ID No : 12.

3. Protéine selon la revendication 1, comprenant une séquence d'acide aminé selon SEQ ID No : 14.

4. Procédé pour la production d'une protéine selon la revendication 1, comprenant les étapes suivantes :
a) production d'un oligonucléotide (gène), qui comprend au minimum les bases 37-954 de la séquence d'ADN de AsnO,
b) mutagenèse dirigée de ce gène, où le codon aspartate des bases 721-723 de AsnO est remplacé par un codon asparagine,
c) clonage de ce gène dans un vecteur d'expression,
d) transformation d'un organisme hôte avec le vecteur d'expression et expression de la protéine recombinée.

5. Procédé selon la revendication 4, **caractérisé en ce que** lors de la mutagenèse dirigée le codon aspartate des bases 721-723 de AsnO est remplacé par le codon asparagine AAC.

6. Procédé selon la revendication 4, **caractérisé en ce que** lors de la mutagenèse dirigée le codon aspartate des bases 721-723 de AsnO est remplacé par le codon asparagine AAT.

7. Procédé selon l'une des revendications 4 à 6, **caractérisé en ce que** le plasmide d'expression est un His-plasmide de fusion.

8. Procédé selon l'une des revendications 4 à 7, **caractérisé en ce que** pQTEV est utilisé comme vecteur.

9. Procédé selon l'une des revendications 4 à 8, **caractérisé en ce que** *Eschericia coli* est utilisé comme organisme hôte.

10. Protéine, susceptible d'être obtenue par un procédé selon l'une des revendications 4 à 9.

11. Utilisation d'une protéine selon la revendication 1 pour la production chimio-enzymatique et énantiosélective de L-fhreo-hydroxyaspartate.


### SEQUENCE LISTING

<110> Philipps-universität Marburg
<120> Protein zur chemoenzymatischen Herstellung von L-threo-Hydroxyaspartat
<130> TM218
<160> 17
<170> PatentIn version 3.3
<210> 1
   <211> 1002
   <212> DNA
   <213> Streptomyces coelicolor
<220>
   <221> AsnO-Wildtyp (DNA)
   <222> (1)..(1002)
   <223> Gene: complement (3587687..3588688) Locus_tag="sco3236"
   Synonym: "SCE29.05c"
<300>
   <301> Bentley,S.D., chater,K.F., cerdeno-Tarraga,A.M., Challis,G.L., et al.
   <302> Complete genome sequence of the model actinomycete Streptomyces coelicolor A3(2)
   <303> Nature
   <304> 417
   <305> 6885
   <306> 141-147
   <307> 2002-05-09
   <308> GenBank / NC_003888
   <309> 2006-04-03
   <313> (1)..(1002)
<400> 1
   atggctgcga atgccgcggg accggcgtcg cgctacgacg tgacgctgga tcagtcggat      60
   gcggaactcg tcgaggagat cgcctggaaa ctcgccacgc aggcgaccgg gcggcccgac     120
   gacgccgaat gggtcgaggc ggccaggaac gcctggcacg cctggccggc gaccctgcgc     180
   cgggacctcg ccggattccg ccgggactcg ggaccggacg gcgcgatcgt gctgcgcggt     240
   ctgcccgtcg actccatggg gctgccgccg accccgcggg tcaacggctc cgtgcagcgc     300
   gaggcctcgc tgggcgccgc cgtgctgctg atgaccgcct gcgggctcgg cgaccccggc     360
   gcgttcctgc cggagaagaa cggcgccctc gtacaggacg tcgtccccgt gccgggcatg     420
   gaggagttcc agggcaacgc cggttcgacc ctgctgacgt tccacaacga gaacgccttc     480
   cacgagcacc gccccgactt cgtgatgctg ctgtgcctgc gcgccgaccc cacgggccgg     540
   gcgggcctgc gcaccgcgtg cgtgcggcgg gtgctgccgc tgctgtccga ctccaccgtg     600
   gacgccctgt gggcaccgga gttccgcacc gcgccgccgc cctccttcca gctgagcggc     660
   cccgaggagg cacccgcacc ggtactcctc ggcgaccgtt cggaccccga cctgcgggtg     720
   gacctggcgg ccaccgagcc ggtgaccgag cgggccgccg aggccctgcg cgaactccag     780
   gcccacttcg acgccaccgc cgtcacccac cgcctcctgc ccggggagct ggcgatcgtg     840
   gacaaccgcg tcaccgtgca cggccgcacc gagttcaccc cccgctacga cggcaccgac     900
   cgctggctcc agcgcacctt cgtgctcacc gacctgcgcc gctcgcgcgc gatgcggccg     960
   gccgacggct acgtgctcgg agcggccccg cagcccgcct ga                        1002
<210> 2
   <211> 333
   <212> PRT
   <213> Streptomyces coelicolor
<220>
   <221> Asno-Wildtyp (Protein)
   <222> (1)..(333)
   <223> Natürlich vorkommende Asparagin-Oxygenase aus Streptomyces coelicolor
<300>
   <301> Bentley,S.D., Chater,K.F., Cerdeno-Tarraga,A.M., challis,G.L., et al.
   <302> Complete genome sequence of the model actinomycete Streptomyces coelicolor A3(2)
   <303> Nature
<304> 417
   <305> 6885
   <306> 141-147
   <307> 2002-05-09
   <308> GenBank / NP_627448
   <309> 2006-04-03
   <313> (1)..(333)
<400> 2
   Met Ala Ala Asn Ala Ala Gly Pro Ala Ser Arg Tyr Asp Val Thr Leu
   1               5                   10                  15
   Asp Gln Ser Asp Ala Glu Leu Val Glu Glu Ile Ala Trp Lys Leu Ala
               20                  25                  30
   Thr Gln Ala Thr Gly Arg Pro Asp Asp Ala Glu Trp Val Glu Ala Ala
           35                  40                  45
   Arg Asn Ala Trp His Ala Trp Pro Ala Thr Leu Arg Arg Asp Leu Ala
       50                  55                  60
   Gly Phe Arg Arg Asp Ser Gly Pro Asp Gly Ala Ile Val Leu Arg Gly
   65                  70                  75                  80
   Leu Pro Val Asp Ser Met Gly Leu Pro Pro Thr Pro Arg Val Asn Gly
                   85                  90                  95
   Ser Val Gln Arg Glu Ala Ser Leu Gly Ala Ala Val Leu Leu Met Thr
               100                 105                 110
   Ala Cys Gly Leu Gly Asp Pro Gly Ala Phe Leu Pro Glu Lys Asn Gly
           115                 120                 125
   Ala Leu Val Gln Asp Val Val Pro Val Pro Gly Met Glu Glu Phe Gln
       130                 135                 140
   Gly Asn Ala Gly Ser Thr Leu Leu Thr Phe His Asn Glu Asn Ala Phe
   145                 150                 155                 160
   His Glu His Arg Pro Asp Phe Val Met Leu Leu Cys Leu Arg Ala Asp
                   165                 170                 175
   Pro Thr Gly Arg Ala Gly Leu Arg Thr Ala Cys Val Arg Arg Val Leu
               180                 185                 190
   Pro Leu Leu Ser Asp Ser Thr Val Asp Ala Leu Trp Ala Pro Glu Phe
           195                 200                 205
   Arg Thr Ala Pro Pro Pro Ser Phe Gln Leu Ser Gly Pro Glu Glu Ala
       210                 215                 220
   Pro Ala Pro Val Leu Leu Gly Asp Arg Ser Asp Pro Asp Leu Arg Val
   225                 230                 235                 240
   Asp Leu Ala Ala Thr Glu Pro Val Thr Glu Arg Ala Ala Glu Ala Leu
                   245                 250                 255
   Arg Glu Leu Gln Ala His Phe Asp Ala Thr Ala Val Thr His Arg Leu
               260                 265                 270
   Leu Pro Gly Glu Leu Ala Ile Val Asp Asn Arg Val Thr Val His Gly
           275                 280                 285
   Arg Thr Glu Phe Thr Pro Arg Tyr Asp Gly Thr Asp Arg Trp Leu Gln
       290                 295                 300
   Arg Thr Phe Val Leu Thr Asp Leu Arg Arg Ser Arg Ala Met Arg Pro
   305                 310                 315                 320
   Ala Asp Gly Tyr Val Leu Gly Ala Ala Pro Gln Pro Ala
                   325                 330
<210> 3
   <211> 1002
   <212> DNA
   <213> Streptomyces coelicolor
<220>
   <221> AsnO D241N (DNA) AAC-Codon
   <222> (1)..(1002)
   <223> Site-directed Mutagensis der DNA der natürlichen Asparagin-Oxgenase AsnO Codon 721-723 (im wildtyp GAC für Asp) wurde gegen AAC (für Asn) ausgetauscht.
<400> 3
   atggctgcga atgccgcggg accggcgtcg cgctacgacg tgacgctgga tcagtcggat      60
   gcggaactcg tcgaggagat cgcctggaaa ctcgccacgc aggcgaccgg gcggcccgac     120
   gacgccgaat gggtcgaggc ggccaggaac gcctggcacg cctggccggc gaccctgcgc     180
   cgggacctcg ccggattccg ccgggactcg ggaccggacg gcgcgatcgt gctgcgcggt     240
   ctgcccgtcg actccatggg gctgccgccg accccgcggg tcaacggctc cgtgcagcgc     300
   gaggcctcgc tgggcgccgc cgtgctgctg atgaccgcct gcgggctcgg cgaccccggc     360
   gcgttcctgc cggagaagaa cggcgccctc gtacaggacg tcgtccccgt gccgggcatg     420
   gaggagttcc agggcaacgc cggttcgacc ctgctgacgt tccacaacga gaacgccttc     480
   cacgagcacc gccccgactt cgtgatgctg ctgtgcctgc gcgccgaccc cacgggccgg     540
   gcgggcctgc gcaccgcgtg cgtgcggcgg gtgctgccgc tgctgtccga ctccaccgtg     600
   gacgccctgt gggcaccgga gttccgcacc gcgccgccgc cctccttcca gctgagcggc     660
   cccgaggagg cacccgcacc ggtactcctc ggcgaccgtt cggaccccga cctgcgggtg     720
   aacctggcgg ccaccgagcc ggtgaccgag cgggccgccg aggccctgcg cgaactccag     780
   gcccacttcg acgccaccgc cgtcacccac cgcctcctgc ccggggagct ggcgatcgtg     840
   gacaaccgcg tcaccgtgca cggccgcacc gagttcaccc cccgctacga cggcaccgac     900
   cgctggctcc agcgcacctt cgtgctcacc gacctgcgcc gctcgcgcgc gatgcggccg     960
   gccgacggct acgtgctcgg agcggccccg cagcccgcct ga                       1002
<210> 4
   <211> 333
   <212> PRT
   <213> Streptomyces coelicolor
<220>
   <221> AsnO D241N (Protein)
   <222> (1)..(333)
   <223> Mutante der natürlichen Asparagin-oxgenase AsnO; Asn-241 wurde gegen Asp-241 ausgetauscht.
<220>
   <221> AsnO D241N (Protein)
   <222> (1)..(333)
   <223> Mutante der natürlichen Asparagin-oxgenase AsnO; Asp-241 wurde gegen Asn-241 ausgetauscht.
<400> 4
   Met Ala Ala Asn Ala Ala Gly Pro Ala Ser Arg Tyr Asp Val Thr Leu
   1               5                   10                  15
   Asp Gln Ser Asp Ala Glu Leu Val Glu Glu Ile Ala Trp Lys Leu Ala
               20                  25                  30
   Thr Gln Ala Thr Gly Arg Pro Asp Asp Ala Glu Trp Val Glu Ala Ala
           35                  40                  45
   Arg Asn Ala Trp His Ala Trp Pro Ala Thr Leu Arg Arg Asp Leu Ala
   50 55 60
   Gly Phe Arg Arg Asp Ser Gly Pro Asp Gly Ala Ile Val Leu Arg Gly
   65 70 75 80
   Leu Pro Val Asp Ser Met Gly Leu Pro Pro Thr Pro Arg Val Asn Gly
   85 90 95
   Ser Val Gln Arg Glu Ala Ser Leu Gly Ala Ala Val Leu Leu Met Thr
   100 105 110
   Ala Cys Gly Leu Gly Asp Pro Gly Ala Phe Leu Pro Glu Lys Asn Gly
   115 120 125
   Ala Leu Val Gln Asp Val Val Pro Val Pro Gly Met Glu Glu Phe Gln
   130 135 140
   Gly Asn Ala Gly Ser Thr Leu Leu Thr Phe His Asn Glu Asn Ala Phe
   145 150 155 160
   His Glu His Arg Pro Asp Phe Val Met Leu Leu Cys Leu Arg Ala Asp
   165 170 175
   Pro Thr Gly Arg Ala Gly Leu Arg Thr Ala Cys Val Arg Arg Val Leu
   180 185 190
   Pro Leu Leu Ser Asp Ser Thr Val Asp Ala Leu Trp Ala Pro Glu Phe
   195 200 205
   Arg Thr Ala Pro Pro Pro Ser Phe Gln Leu Ser Gly Pro Glu Glu Ala
   210 215 220
   Pro Ala Pro Val Leu Leu Gly Asp Arg Ser Asp Pro Asp Leu Arg Val
   225 230 235 240
   Asn Leu Ala Ala Thr Glu Pro Val Thr Glu Arg Ala Ala Glu Ala Leu
   245 250 255
   Arg Glu Leu Gln Ala His Phe Asp Ala Thr Ala Val Thr His Arg Leu
   260 265 270
   Leu Pro Gly Glu Leu Ala Ile Val Asp Asn Arg Val Thr Val His Gly
   275 280 285
   Arg Thr Glu Phe Thr Pro Arg Tyr Asp Gly Thr Asp Arg Trp Leu Gln
   290 295 300
   Arg Thr Phe Val Leu Thr Asp Leu Arg Arg Ser Arg Ala Met Arg Pro
   305 310 315 320
   Ala Asp Gly Tyr Val Leu Gly Ala Ala Pro Gln Pro Ala
   325 330
<210> 5
   <211> 35
   <212> DNA
   <213> streptomyces coelicolor
<220>
   <221> oligonukleotidprimer 1
   <222> (1)..(35)
   <223> synthetischer oligonukleotidprimer (operon) für gerichtete Mutagenese des Asno-Wildtyps. Austausch von GAC (wildtyp) gegen AAC (AsnO D241N).
<400> 5
   ccccgacctg cgggtgaacc tggcggccac cgagc 35
<210> 6
   <211> 35
   <212> DNA
   <213> Streptomyces coelicolor
<220>
   <221> 2oligonukleotidprimer
   <222> (1)..(35)
   <223> synthetischer oligonukleotidprimer (operon) für gerichtete Mutagenese des Asno-Wildtyps.
   Reverser Primer: Austausch von GTC (Wildtyp) gegen.GTT (Asno D241N)
<400> 6
   gctcggtggc cgccaggttc acccgcaggt cgggg 35
<210> 7
   <211> 1074
   <212> DNA
   <213> streptomyces coelicolor
<220>
   <221> His7-Fusion von AsnO D241N (DNA)
   <222> (1)..(1074)
   <223> His7-Fusionsinsert für die Expression von AsnO D241N
<400> 7
   atgaaacatc accatcacca tcaccatagc gattacgaca tccccactac tgagaatctt 60
   tattttcagg gatccgctgc gaatgccgcg ggaccggcgt cgcgctacga cgtgacgctg 120
   gatcagtcgg atgcggaact cgtcgaggag atcgcctgga aactcgccac gcaggcgacc 180
   gggcggcccg acgacgccga atgggtcgag gcggccagga acgcctggca cgcctggccg 240
   gcgaccctgc gccgggacct cgccggattc cgccgggact cgggaccgga cggcgcgatc 300
   gtgctgcgcg gtctgcccgt cgactccatg gggctgccgc cgaccccgcg ggtcaacggc 360
   tccgtgcagc gcgaggcctc gctgggcgcc gccgtgctgc tgatgaccgc ctgcgggctc 420
   ggcgaccccg gcgcgttcct gccggagaag aacggcgccc tcgtacagga cgtcgtcccc 480
   gtgccgggca tggaggagtt ccagggcaac gccggttcga ccctgctgac gttccacaac    540
   gagaacgcct tccacgagca ccgccccgac ttcgtgatgc tgctgtgcct gcgcgccgac    600
   cccacgggcc gggcgggcct gcgcaccgcg tgcgtgcggc gggtgctgcc gctgctgtcc    660
   gactccaccg tggacgccct gtgggcaccg gagttccgca ccgcgccgcc gccctccttc    720
   cagctgagcg gccccgagga ggcacccgca ccggtactcc tcggcgaccg ttcggacccc    780
   gacctgcggg tgaacctggc ggccaccgag ccggtgaccg agcgggccgc cgaggccctg    840
   cgcgaactcc aggcccactt cgacgccacc gccgtcaccc accgcctcct gcccggggag    900
   ctggcgatcg tggacaaccg cgtcaccgtg cacggccgca ccgagttcac cccccgctac    960
   gacggcaccg accgctggct ccagcgcacc ttcgtgctca ccgacctgcg ccgctcgcgc   1020
   gcgatgcggc cggccgacgg ctacgtgctc ggagcggccc cgcagcccgc ctga         1074
<210> 8
   <211> 357
   <212> PRT
   <213> Streptomyces coelicolor
<220>
   <221> AsnO D241N His7-Tag (Aminosäuresequenz)
   <222> (1)..(357)
   <223> sequenz des exprimierten Proteins nach Aufreinigung über Ni-NTA-Affinitätschromatographie
<400> 8
   Met Lys His His His His His His His Ser Asp Tyr Asp Ile Pro Thr
   1               5                   10                  15
   Thr Glu Asn Leu Tyr Phe Gln Gly Ser Ala Ala Asn Ala Ala Gly Pro
               20                  25                  30
   Ala Ser Arg Tyr Asp Val Thr Leu Asp Gln Ser Asp Ala Glu Leu Val
           35                  40                  45
   Glu Glu Ile Ala Trp Lys Leu Ala Thr Gln Ala Thr Gly Arg Pro Asp
       50                  55                  60
   Asp Ala Glu Trp Val Glu Ala Ala Arg Asn Ala Trp His Ala Trp Pro
   65                  70                  75                  80
   Ala Thr Leu Arg Arg Asp Leu Ala Gly Phe Arg Arg Asp Ser Gly Pro
                   85                  90                  95
   Asp Gly Ala Ile Val Leu Arg Gly Leu Pro Val Asp Ser Met Gly Leu
               100                 105                 110
   Pro Pro Thr Pro Arg Val Asn Gly Ser Val Gln Arg Glu Ala Ser Leu
           115                 120                 125
   Gly Ala Ala Val Leu Leu Met Thr Ala Cys Gly Leu Gly Asp Pro Gly
       130                 135                 140
   Ala Phe Leu Pro Glu Lys Asn Gly Ala Leu Val Gln Asp Val Val Pro
   145                 150                 155                 160
   Val Pro Gly Met Glu Glu Phe Gln Gly Asn Ala Gly Ser Thr Leu Leu
                   165                 170                 175
   Thr Phe His Asn Glu Asn Ala Phe His Glu His Arg Pro Asp Phe Val
               180                 185                 190
   Met Leu Leu Cys Leu Arg Ala Asp Pro Thr Gly Arg Ala Gly Leu Arg
           195                 200                 205
   Thr Ala Cys Val Arg Arg Val Leu Pro Leu Leu Ser Asp Ser Thr Val
       210                 215                 220
   Asp Ala Leu Trp Ala Pro Glu Phe Arg Thr Ala Pro Pro Pro Ser Phe
   225                 230                 235                 240
   Gln Leu Ser Gly Pro Glu Glu Ala Pro Ala Pro Val Leu Leu Gly Asp
                   245                 250                 255
   Arg Ser Asp Pro Asp Leu Arg Val Asn Leu Ala Ala Thr Glu Pro Val
               260                 265                 270
   Thr Glu Arg Ala Ala Glu Ala Leu Arg Glu Leu Gln Ala His Phe Asp
           275                 280                 285
   Ala Thr Ala Val Thr His Arg Leu Leu Pro Gly Glu Leu Ala Ile Val
       290                 295                 300
   Asp Asn Arg Val Thr Val His Gly Arg Thr Glu Phe Thr Pro Arg Tyr
   305                 310                 315                 320
   Asp Gly Thr Asp Arg Trp Leu Gln Arg Thr Phe Val Leu Thr Asp Leu
                   325                 330                 335
   Arg Arg Ser Arg Ala Met Arg Pro Ala Asp Gly Tyr Val Leu Gly Ala
               340                 345                 350
   Ala Pro Gln Pro Ala
           355
<210> 9
   <211> 1002
   <212> DNA
   <213> streptomyces coelicolor
<220>
   <221> AsnO D241N und m1S (DNA)
   <222> (1)..(1002)
   <223> Basensequenz von AsnO D241N ohne Klonierungsartefakte; diese sind der His7-Tag, eine kurze Verbindungsregion und die "Tobacco Etch Virus Protease" Erkennungsstelle
<400> 9
   tccgctgcga atgccgcggg accggcgtcg cgctacgacg tgacgctgga tcagtcggat      60
   gcggaactcg tcgaggagat cgcctggaaa ctcgccacgc aggcgaccgg gcggcccgac     120
   gacgccgaat gggtcgaggc ggccaggaac gcctggcacg cctggccggc gaccctgcgc     180
   cgggacctcg ccggattccg ccgggactcg ggaccggacg gcgcgatcgt gctgcgcggt     240
   ctgcccgtcg actccatggg gctgccgccg accccgcggg tcaacggctc cgtgcagcgc     300
   gaggcctcgc tgggcgccgc cgtgctgctg atgaccgcct gcgggctcgg cgaccccggc     360
   gcgttcctgc cggagaagaa cggcgccctc gtacaggacg tcgtccccgt gccgggcatg     420
   gaggagttcc agggcaacgc cggttcgacc ctgctgacgt tccacaacga gaacgccttc     480
   cacgagcacc gccccgactt cgtgatgctg ctgtgcctgc gcgccgaccc cacgggccgg     540
   gcgggcctgc gcaccgcgtg cgtgcggcgg gtgctgccgc tgctgtccga ctccaccgtg     600
   gacgccctgt gggcaccgga gttccgcacc gcgccgccgc cctccttcca gctgagcggc     660
   cccgaggagg cacccgcacc ggtactcctc ggcgaccgtt cggaccccga cctgcgggtg     720
   aacctggcgg ccaccgagcc ggtgaccgag cgggccgccg aggccctgcg cgaactccag     780
   gcccacttcg acgccaccgc cgtcacccac cgcctcctgc ccggggagct ggcgatcgtg     840
   gacaaccgcg tcaccgtgca cggccgcacc gagttcaccc cccgctacga cggcaccgac     900
   cgctggctcc agcgcacctt cgtgctcacc gacctgcgcc gctcgcgcgc gatgcggccg     960
   gccgacggct acgtgctcgg agcggccccg cagcccgcct ga                       1002
<210> 10
   <211> 333
   <212> PRT
   <213> Streptomyces coelicolor
<220>
   <221> AsnO D241 N und M1S (Aminosäuresequenz)
   <222> (1)..(333)
   <223> Mutante der natürlichen Asparagin-Oxgenase AsnO:
   Aminosäuresequenz von AsnO D241N ohne Klonierungsartefakte; diese sind der His7-Tag, eine kurze Verbindungsregion und die "Tobacco Etch Virus Protease" Erkennungsstelle
<400> 10
   Ser Ala Ala Asn Ala Ala Gly Pro Ala Ser Arg Tyr Asp Val Thr Leu
   1               5                   10                  15
   Asp Gln Ser Asp Ala Glu Leu Val Glu Glu Ile Ala Trp Lys Leu Ala
               20                  25                  30
   Thr Gln Ala Thr Gly Arg Pro Asp Asp Ala Glu Trp Val Glu Ala Ala
           35                  40                  45
   Arg Asn Ala Trp His Ala Trp Pro Ala Thr Leu Arg Arg Asp Leu Ala
       50                  55                  60
   Gly Phe Arg Arg Asp Ser Gly Pro Asp Gly Ala Ile Val Leu Arg Gly
   65                  70                  75                  80
   Leu Pro Val Asp Ser Met Gly Leu Pro Pro Thr Pro Arg Val Asn Gly
                   85                  90                  95
   Ser Val Gln Arg Glu Ala Ser Leu Gly Ala Ala Val Leu Leu Met Thr
               100                 105                 110
   Ala Cys Gly Leu Gly Asp Pro Gly Ala Phe Leu Pro Glu Lys Asn Gly
           115                 120                 125
   Ala Leu Val Gln Asp Val Val Pro Val Pro Gly Met Glu Glu Phe Gln
       130                 135                 140
   Gly Asn Ala Gly Ser Thr Leu Leu Thr Phe His Asn Glu Asn Ala Phe
   145                 150                 155                 160
   His Glu His Arg Pro Asp Phe Val Met Leu Leu Cys Leu Arg Ala Asp
                   165                 170                 175
   Pro Thr Gly Arg Ala Gly Leu Arg Thr Ala Cys Val Arg Arg Val Leu
               180                 185                 190
   Pro Leu Leu Ser Asp Ser Thr Val Asp Ala Leu Trp Ala Pro Glu Phe
           195                 200                 205
   Arg Thr Ala Pro Pro Pro Ser Phe Gln Leu Ser Gly Pro Glu Glu Ala
       210                 215                 220
   Pro Ala Pro Val Leu Leu Gly Asp Arg Ser Asp Pro Asp Leu Arg Val
   225                 230                 235                 240
   Asn Leu Ala Ala Thr Glu Pro Val Thr Glu Arg Ala Ala Glu Ala Leu
                   245                 250                 255
   Arg Glu Leu Gln Ala His Phe Asp Ala Thr Ala Val Thr His Arg Leu
               260                 265                 270
   Leu Pro Gly Glu Leu Ala Ile Val Asp Asn Arg Val Thr Val His Gly
           275                 280                 285
   Arg Thr Glu Phe Thr Pro Arg Tyr Asp Gly Thr Asp Arg Trp Leu Gln
       290                 295                 300
   Arg Thr Phe Val Leu Thr Asp Leu Arg Arg Ser Arg Ala Met Arg Pro
   305                 310                 315                 320
   Ala Asp Gly Tyr Val Leu Gly Ala Ala Pro Gln Pro Ala
                   325                     330
<210> 11
   <211> 918
   <212> DNA
   <213> Streptomyces coelicolor
<220>
   <221> Nukleotidsequenz 37-954 von AsnO D241N
   <222> (1)..(918)
   <223> Gerichtete Mutagenese der DNA der natürlichen Asparagin-oxgenase AsnO
   Codon 721-723 (im Wildtyp GAC für Asp) wurde gegen AAC (für Asn) ausgetauscht.
Basen 37-954
<400> 11
   gacgtgacgc tggatcagtc ggatgcggaa ctcgtcgagg agatcgcctg gaaactcgcc    60
   acgcaggcga ccgggcggcc cgacgacgcc gaatgggtcg aggcggccag gaacgcctgg   120
   cacgcctggc cggcgaccct gcgccgggac ctcgccggat tccgccggga ctcgggaccg   180
   gacggcgcga tcgtgctgcg cggtctgccc gtcgactcca tggggctgcc gccgaccccg   240
   cgggtcaacg gctccgtgca gcgcgaggcc tcgctgggcg ccgccgtgct gctgatgacc   300
   gcctgcgggc tcggcgaccc cggcgcgttc ctgccggaga agaacggcgc cctcgtacag   360
   gacgtcgtcc ccgtgccggg catggaggag ttccagggca acgccggttc gaccctgctg   420
   acgttccaca acgagaacgc cttccacgag caccgccccg acttcgtgat gctgctgtgc   480
   ctgcgcgccg accccacggg ccgggcgggc ctgcgcaccg cgtgcgtgcg gcgggtgctg   540
   ccgctgctgt ccgactccac cgtggacgcc ctgtgggcac cggagttccg caccgcgccg   600
   ccgccctcct tccagctgag cggccccgag gaggcacccg caccggtact cctcggcgac   660
   cgttcggacc ccgacctgcg ggtgaacctg gcggccaccg agccggtgac cgagcgggcc   720
   gccgaggccc tgcgcgaact ccaggcccac ttcgacgcca ccgccgtcac ccaccgcctc   780
   ctgcccgggg agctggcgat cgtggacaac cgcgtcaccg tgcacggccg caccgagttc   840
   accccccgct acgacggcac cgaccgctgg ctccagcgca ccttcgtgct caccgacctg   900
   cgccgctcgc gcgcgatg                                                 918
<210> 12
   <211> 306
   <212> PRT
   <213> Streptomyces coelicolor
<220>
   <221> Aminosäuresequenz 13-318 von AsnO D241N
   <222> (1)..(306)
   <223> Mutante der natürlichen Asparagin-Oxgenase AsnO; Asp-241 wurde gegen Asn-241 ausgetauscht
   Gezeigt sind Aminosäuren 13-318
<400> 12
   Asp Val Thr Leu Asp Gln Ser Asp Ala Glu Leu Val Glu Glu Ile Ala
   1 5 10 15
   Trp Lys Leu Ala Thr Gln Ala Thr Gly Arg Pro Asp Asp Ala Glu Trp
   20 25 30
   Val Glu Ala Ala Arg Asn Ala Trp His Ala Trp Pro Ala Thr Leu Arg
   35 40 45
   Arg Asp Leu Ala Gly Phe Arg Arg Asp Ser Gly Pro Asp Gly Ala Ile
   50 55 60
   Val Leu Arg Gly Leu Pro Val Asp Ser Met Gly Leu Pro Pro Thr Pro
   65 70 75 80
   Arg Val Asn Gly Ser Val Gln Arg Glu Ala Ser Leu Gly Ala Ala Val
   85 90 95
   Leu Leu Met Thr Ala Cys Gly Leu Gly Asp Pro Gly Ala Phe Leu Pro
   100 105 110
   Glu Lys Asn Gly Ala Leu Val Gln Asp Val Val Pro Val Pro Gly Met
   115 120 125
   Glu Glu Phe Gln Gly Asn Ala Gly Ser Thr Leu Leu Thr Phe His Asn
   130 135 140
   Glu Asn Ala Phe His Glu His Arg Pro Asp Phe Val Met Leu Leu Cys
   145 150 155 160
   Leu Arg Ala Asp Pro Thr Gly Arg Ala Gly Leu Arg Thr Ala Cys Val
   165 170 175
   Arg Arg Val Leu Pro Leu Leu Ser Asp Ser Thr Val Asp Ala Leu Trp
   180 185 190
   Ala Pro Glu Phe Arg Thr Ala Pro Pro Pro Ser Phe Gln Leu Ser Gly
   195 200 205
   Pro Glu Glu Ala Pro Ala Pro Val Leu Leu Gly Asp Arg Ser Asp Pro
   210 215 220
   Asp Leu Arg Val Asn Leu Ala Ala Thr Glu Pro Val Thr Glu Arg Ala
   225 230 235 240
   Ala Glu Ala Leu Arg Glu Leu Gln Ala His Phe Asp Ala Thr Ala Val
   245 250 255
   Thr His Arg Leu Leu Pro Gly Glu Leu Ala Ile Val Asp Asn Arg Val
               260                 265                 270
   Thr Val His Gly Arg Thr Glu Phe Thr Pro Arg Tyr Asp Gly Thr Asp
           275                 280                 285
   Arg Trp Leu Gln Arg Thr Phe Val Leu Thr Asp Leu Arg Arg Ser Arg
       290                 295                 300
   Ala Met
   305
<210> 13
   <211> 996
   <212> DNA
   <213> Streptomyces coelicolor
<220>
   <221> Nukleotidsequenz 4-999 von AsnO D241N - AAC-Codon
   <222> (1)..(996)
   <223> Site-directed Mutagensis der DNA der natürlichen Asparagin-Oxgenase AsnO
   Codon 721-723 (im Wildtyp GAC für Asp) wurde gegen AAC (für Asn) ausgetauscht.
   Basen 4-999
<400> 13
   gctgcgaatg ccgcgggacc ggcgtcgcgc tacgacgtga cgctggatca gtcggatgcg     60
   gaactcgtcg aggagatcgc ctggaaactc gccacgcagg cgaccgggcg gcccgacgac    120
   gccgaatggg tcgaggcggc caggaacgcc tggcacgcct ggccggcgac cctgcgccgg    180
   gacctcgccg gattccgccg ggactcggga ccggacggcg cgatcgtgct gcgcggtctg    240
   cccgtcgact ccatggggct gccgccgacc ccgcgggtca acggctccgt gcagcgcgag    300
   gcctcgctgg gcgccgccgt gctgctgatg accgcctgcg ggctcggcga ccccggcgcg    360
   ttcctgccgg agaagaacgg cgccctcgta caggacgtcg tccccgtgcc gggcatggag    420
   gagttccagg gcaacgccgg ttcgaccctg ctgacgttcc acaacgagaa cgccttccac    480
   gagcaccgcc ccgacttcgt gatgctgctg tgcctgcgcg ccgaccccac gggccgggcg    540
   ggcctgcgca ccgcgtgcgt gcggcgggtg ctgccgctgc tgtccgactc caccgtggac    600
   gccctgtggg caccggagtt ccgcaccgcg ccgccgccct ccttccagct gagcggcccc    660
   gaggaggcac ccgcaccggt actcctcggc gaccgttcgg accccgacct gcgggtgaac    720
   ctggcggcca ccgagccggt gaccgagcgg gccgccgagg ccctgcgcga actccaggcc    780
   cacttcgacg ccaccgccgt cacccaccgc ctcctgcccg gggagctggc gatcgtggac    840
   aaccgcgtca ccgtgcacgg ccgcaccgag ttcacccccc gctacgacgg caccgaccgc    900
   tggctccagc gcaccttcgt gctcaccgac ctgcgccgct cgcgcgcgat gcggccggcc    960
   gacggctacg tgctcggagc ggccccgcag cccgcc                              996
<210> 14
   <211> 332
   <212> PRT
   <213> streptomyces coelicolor
<220>
   <221> Aminosäuresequenz 2-333 von AsnO D241N
   <222> (1)..(332)
   <223> Mutante der natürlichen Asparagin-oxgenase AsnO; Asp-241 wurde gegen Asn-241 ausgetauscht
   Gezeigt sind Aminosäuren 2-333
<400> 14
   Ala Ala Asn Ala Ala Gly Pro Ala Ser Arg Tyr Asp Val Thr Leu Asp
   1               5                   10                  15
   Gln Ser Asp Ala Glu Leu Val Glu Glu Ile Ala Trp Lys Leu Ala Thr
               20                  25                  30
   Gln Ala Thr Gly Arg Pro Asp Asp Ala Glu Trp Val Glu Ala Ala Arg
           35                  40                  45
   Asn Ala Trp His Ala Trp Pro Ala Thr Leu Arg Arg Asp Leu Ala Gly
       50                  55                  60
   Phe Arg Arg Asp Ser Gly Pro Asp Gly Ala Ile Val Leu Arg Gly Leu
   65                  70                  75                  80
   Pro Val Asp Ser Met Gly Leu Pro Pro Thr Pro Arg Val Asn Gly Ser
                   85                  90                  95
   Val Gln Arg Glu Ala Ser Leu Gly Ala Ala Val Leu Leu Met Thr Ala
               100                 105                 110
   Cys Gly Leu Gly Asp Pro Gly Ala Phe Leu Pro Glu Lys Asn Gly Ala
           115                 120                 125
   Leu Val Gln Asp Val Val Pro Val Pro Gly Met Glu Glu Phe Gln Gly
       130                 135                 140
   Asn Ala Gly Ser Thr Leu Leu Thr Phe His Asn Glu Asn Ala Phe His
   145                 150                 155                 160
   Glu His Arg Pro Asp Phe Val Met Leu Leu Cys Leu Arg Ala Asp Pro
                   165                 170                 175
   Thr Gly Arg Ala Gly Leu Arg Thr Ala Cys Val Arg Arg Val Leu Pro
               180                 185                 190
   Leu Leu Ser Asp Ser Thr Val Asp Ala Leu Trp Ala Pro Glu Phe Arg
           195                 200                 205
   Thr Ala Pro Pro Pro Ser Phe Gln Leu Ser Gly Pro Glu Glu Ala Pro
       210                 215                 220
   Ala Pro Val Leu Leu Gly Asp Arg Ser Asp Pro Asp Leu Arg Val Asn
   225                 230                 235                 240
   Leu Ala Ala Thr Glu Pro Val Thr Glu Arg Ala Ala Glu Ala Leu Arg
                   245                 250                 255
   Glu Leu Gln Ala His Phe Asp Ala Thr Ala Val Thr His Arg Leu Leu
               260                 265                 270
   Pro Gly Glu Leu Ala Ile Val Asp Asn Arg Val Thr Val His Gly Arg
           275                 280                 285
   Thr Glu Phe Thr Pro Arg Tyr Asp Gly Thr Asp Arg Trp Leu Gln Arg
       290                 295                 300
   Thr Phe Val Leu Thr Asp Leu Arg Arg Ser Arg Ala Met Arg Pro Ala
   305                 310                 315                 320
   Asp Gly Tyr Val Leu Gly Ala Ala Pro Gln Pro Ala
                   325                 330
<210> 15
   <211> 1002
   <212> DNA
   <213> Streptomyces coelicolor
<220>
   <221> AsnO D241N (DNA) - AAT-Codon
   <222> (1)..(1002)
   <223> Gerichtete Mutagenese der DNA der natürlichen Asparagin-Oxgenase AsnO
   Codon 721-723 (im Wildtyp GAC für Asn) wurde gegen AAT (für Asn) ausgetauscht.
<400> 15
   atggctgcga atgccgcggg accggcgtcg cgctacgacg tgacgctgga tcagtcggat      60
   gcggaactcg tcgaggagat cgcctggaaa ctcgccacgc aggcgaccgg gcggcccgac     120
   gacgccgaat gggtcgaggc ggccaggaac gcctggcacg cctggccggc gaccctgcgc     180
   cgggacctcg ccggattccg ccgggactcg ggaccggacg gcgcgatcgt gctgcgcggt     240
   ctgcccgtcg actccatggg gctgccgccg accccgcggg tcaacggctc cgtgcagcgc     300
   gaggcctcgc tgggcgccgc cgtgctgctg atgaccgcct gcgggctcgg cgaccccggc     360
   gcgttcctgc cggagaagaa cggcgccctc gtacaggacg tcgtccccgt gccgggcatg     420
   gaggagttcc agggcaacgc cggttcgacc ctgctgacgt tccacaacga gaacgccttc     480
   cacgagcacc gccccgactt cgtgatgctg ctgtgcctgc gcgccgaccc cacgggccgg     540
   gcgggcctgc gcaccgcgtg cgtgcggcgg gtgctgccgc tgctgtccga ctccaccgtg 600
   gacgccctgt gggcaccgga gttccgcacc gcgccgccgc cctccttcca gctgagcggc 660
   cccgaggagg cacccgcacc ggtactcctc ggcgaccgtt cggaccccga cctgcgggtg 720
   aatctggcgg ccaccgagcc ggtgaccgag cgggccgccg aggccctgcg cgaactccag 780
   gcccacttcg acgccaccgc cgtcacccac cgcctcctgc ccggggagct ggcgatcgtg 840
   gacaaccgcg tcaccgtgca cggccgcacc gagttcaccc cccgctacga cggcaccgac 900
   cgctggctcc agcgcacctt cgtgctcacc gacctgcgcc gctcgcgcgc gatgcggccg 960
   gccgacggct acgtgctcgg agcggccccg cagcccgcct ga 1002
<210> 16
   <211> 996
   <212> DNA
   <213> Streptomyces coelicolor
<220>
   <221> Nukleotidsequenz 4-999 von AsnO D241N - AAT-Codon
   <222> (1)..(996)
   <223> Gerichtete Mutagenese der DNA der natürlichen Asparagin-oxgenase AsnO
   codon 721-723 (im wildtyp GAC für Asn) wurde gegen AAT (für Asn) ausgetauscht.
   Basen 4-999
<400> 16
   gctgcgaatg ccgcgggacc ggcgtcgcgc tacgacgtga cgctggatca gtcggatgcg 60
   gaactcgtcg aggagatcgc ctggaaactc gccacgcagg cgaccgggcg gcccgacgac 120
   gccgaatggg tcgaggcggc caggaacgcc tggcacgcct ggccggcgac cctgcgccgg 180
   gacctcgccg gattccgccg ggactcggga ccggacggcg cgatcgtgct gcgcggtctg 240
   cccgtcgact ccatggggct gccgccgacc ccgcgggtca acggctccgt gcagcgcgag 300
   gcctcgctgg gcgccgccgt gctgctgatg accgcctgcg ggctcggcga ccccggcgcg 360
   ttcctgccgg agaagaacgg cgccctcgta caggacgtcg tccccgtgcc gggcatggag 420
   gagttccagg gcaacgccgg ttcgaccctg ctgacgttcc acaacgagaa cgccttccac 480
   gagcaccgcc ccgacttcgt gatgctgctg tgcctgcgcg ccgaccccac gggccgggcg 540
   ggcctgcgca ccgcgtgcgt gcggcgggtg ctgccgctgc tgtccgactc caccgtggac 600
   gccctgtggg caccggagtt ccgcaccgcg ccgccgccct ccttccagct gagcggcccc 660
   gaggaggcac ccgcaccggt actcctcggc gaccgttcgg accccgacct gcgggtgaat 720
   ctggcggcca ccgagccggt gaccgagcgg gccgccgagg ccctgcgcga actccaggcc 780
   cacttcgacg ccaccgccgt cacccaccgc ctcctgcccg gggagctggc gatcgtggac 840
   aaccgcgtca ccgtgcacgg ccgcaccgag ttcacccccc gctacgacgg caccgaccgc 900
   tggctccagc gcaccttcgt gctcaccgac ctgcgccgct cgcgcgcgat gcggccggcc 960
   gacggctacg tgctcggagc ggccccgcag cccgcc 996
<210> 17
   <211> 4803
   <212> DNA
   <213> Klonierungsvektor pQTEV
<300>
   <308> GenBank / AY_243506
   <309> 2002-02-25
   <313> (1)..(4803)
<400> 17
   ctcgagaaat cataaaaaat ttatttgctt tgtgagcgga taacaattat aatagattca      60
   attgtgagcg gataacaatt tcacacagaa ttcattaaag aggagaaatt aactatgaaa     120
   catcaccatc accatcacca tagcgattac gacatcccca ctactgagaa tctttatttt     180
   cagggatccg ggtcgactgt tgatagatct cggtaccgcg gccgctcgac ctgcagccaa     240
   gcttaattag ctgagcttgg actcctgttg atagatccag taatgacctc agaactccat     300
   ctggatttgt tcagaacgct cggttgccgc cgggcgtttt ttattggtga gaatccaagc     360
   tagcttggcg agattttcag gagctaagga agctaaaatg gagaaaaaaa tcactggata     420
   taccaccgtt gatatatccc aatggcatcg taaagaacat tttgaggcat ttcagtcagt     480
   tgctcaatgt acctataacc agaccgttca gctggatatt acggcctttt taaagaccgt     540
   aaagaaaaat aagcacaagt tttatccggc ctttattcac attcttgccc gcctgatgaa     600
   tgctcatccg gaatttcgta tggcaatgaa agacggtgag ctggtgatat gggatagtgt     660
   tcacccttgt tacaccgttt tccatgagca aactgaaacg ttttcatcgc tctggagtga     720
   ataccacgac gatttccggc agtttctaca catatattcg caagatgtgg cgtgttacgg     780
   tgaaaacctg gcctatttcc ctaaagggtt tattgagaat atgtttttcg tctcagccaa     840
   tccctgggtg agtttcacca gttttgattt aaacgtggcc aatatggaca acttcttcgc     900
   ccccgttttc accatgggca aatattatac gcaaggcgac aaggtgctga tgccgctggc     960
   gattcaggtt catcatgccg tttgtgatgg cttccatgtc ggcagaatgc ttaatgaatt    1020
   acaacagtac tgcgatgagt ggcagggcgg ggcgtaattt ttttaaggca gttattggtg    1080
   cccttaaacg cctggggtaa tgactctcta gcttgaggca tcaaataaaa cgaaaggctc    1140
   agtcgaaaga ctgggccttt cgttttatct gttgtttgtc ggtgaacgct ctcctgagta    1200
   ggacaaatcc gccctctaga ttacgtgcag tcgatgataa gctgtcaaac atgagaattg    1260
   tgcctaatga gtgagctaac ttacattaat tgcgttgcgc tcactgcccg ctttccagtc    1320
   gggaaacctg tcgtgccagc tgcattaatg aatcggccaa cgcgcgggga gaggcggttt    1380
   gcgtattggg cgccagggtg gtttttcttt tcaccagtga gacgggcaac agctgattgc    1440
   ccttcaccgc ctggccctga gagagttgca gcaagcggtc cacgctggtt tgccccagca    1500
   ggcgaaaatc ctgtttgatg gtggttaacg gcgggatata acatgagctg tcttcggtat    1560
   cgtcgtatcc cactaccgag atatccgcac caacgcgcag cccggactcg gtaatggcgc    1620
   gcattgcgcc cagcgccatc tgatcgttgg caaccagcat cgcagtggga acgatgccct    1680
   cattcagcat ttgcatggtt tgttgaaaac cggacatggc actccagtcg ccttcccgtt 1740
   ccgctatcgg ctgaatttga ttgcgagtga gatatttatg ccagccagcc agacgcagac 1800
   gcgccgagac agaacttaat gggcccgcta acagcgcgat ttgctggtga cccaatgcga 1860
   ccagatgctc cacgcccagt cgcgtaccgt cttcatggga gaaaataata ctgttgatgg 1920
   gtgtctggtc agagacatca agaaataacg ccggaacatt agtgcaggca gcttccacag 1980
   caatggcatc ctggtcatcc agcggatagt taatgatcag cccactgacg cgttgcgcga 2040
   gaagattgtg caccgccgct ttacaggctt cgacgccgct tcgttctacc atcgacacca 2100
   ccacgctggc acccagttga tcggcgcgag atttaatcgc cgcgacaatt tgcgacggcg 2160
   cgtgcagggc cagactggag gtggcaacgc caatcagcaa cgactgtttg cccgccagtt 2220
   gttgtgccac gcggttggga atgtaattca gctccgccat cgccgcttcc actttttccc 2280
   gcgttttcgc agaaacgtgg ctggcctggt tcaccacgcg ggaaacggtc tgataagaga 2340
   caccggcata ctctgcgaca tcgtataacg ttactggttt cacattcacc accctgaatt 2400
   gactctcttc cgggcgctat catgccatac cgcgaaaggt tttgcaccat tcgatggtgt 2460
   cggaatttcg ggcagcgttg ggtcctggcc acgggtgcgc atgatctaga gctgcctcgc 2520
   gcgtttcggt gatgacggtg aaaacctctg acacatgcag ctcccggaga cggtcacagc 2580
   ttgtctgtaa gcggatgccg ggagcagaca agcccgtcag ggcgcgtcag cgggtgttgg 2640
   cgggtgtcgg ggcgcagcca tgacccagtc acgtagcgat agcggagtgt atactggctt 2700
   aactatgcgg catcagagca gattgtactg agagtgcacc acatgcggtg tgaaataccg 2760
   cacagatgcg taaggagaaa ataccgcatc aggcgctctt ccgcttcctc gctcactgac 2820
   tcgctgcgct cggtcgttcg gctgcggcga gcggtatcag ctcactcaaa ggcggtaata 2880
   cggttatcca cagaatcagg ggataacgca ggaaagaaca tgtgagcaaa aggccagcaa 2940
   aaggccagga accgtaaaaa ggccgcgttg ctggcgtttt tccataggct ccgcccccct 3000
   gacgagcatc acaaaaatcg acgctcaagt cagaggtggc gaaacccgac aggactataa 3060
   agataccagg cgtttccccc tggaagctcc ctcgtgcgct ctcctgttcc gaccctgccg 3120
   cttaccggat acctgtccgc ctttctccct tcgggaagcg tggcgctttc tcatagctca 3180
   cgctgtaggt atctcagttc ggtgtaggtc gttcgctcca agctgggctg tgtgcacgaa 3240
   ccccccgttc agcccgaccg ctgcgcctta tccggtaact atcgtcttga gtccaacccg 3300
   gtaagacacg acttatcgcc actggcagca gccactggta acaggattag cagagcgagg 3360
   tatgtaggcg gtgctacaga gttcttgaag tggtggccta actacggcta cactagaagg 3420
   acagtatttg gtatctgcgc tctgctgaag ccagttacct tcggaaaaag agttggtagc 3480
   tcttgatccg gcaaacaaac caccgctggt agcggtggtt tttttgtttg caagcagcag 3540
   attacgcgca gaaaaaaagg atctcaagaa gatcctttga tcttttctac ggggtctgac 3600
   gctcagtgga acgaaaactc acgttaaggg attttggtca tgagattatc aaaaaggatc 3660
   ttcacctaga tccttttaaa ttaaaaatga agttttaaat caatctaaag tatatatgag 3720
   taaacttggt ctgacagtta ccaatgctta atcagtgagg cacctatctc agcgatctgt   3780
   ctatttcgtt catccatagt tgcctgactc cccgtcgtgt agataactac gatacgggag   3840
   ggcttaccat ctggccccag tgctgcaatg ataccgcgag acccacgctc accggctcca   3900
   gatttatcag caataaacca gccagccgga agggccgagc gcagaagtgg tcctgcaact   3960
   ttatccgcct ccatccagtc tattaattgt tgccgggaag ctagagtaag tagttcgcca   4020
   gttaatagtt tgcgcaacgt tgttgccatt gctacaggca tcgtggtgtc acgctcgtcg   4080
   tttggtatgg cttcattcag ctccggttcc caacgatcaa ggcgagttac atgatccccc   4140
   atgttgtgca aaaaagcggt tagctccttc ggtcctccga tcgttgtcag aagtaagttg   4200
   gccgcagtgt tatcactcat ggttatggca gcactgcata attctcttac tgtcatgcca   4260
   tccgtaagat gcttttctgt gactggtgag tactcaacca agtcattctg agaatagtgt   4320
   atgcggcgac cgagttgctc ttgcccggcg tcaatacggg ataataccgc gccacatagc   4380
   agaactttaa aagtgctcat cattggaaaa cgttcttcgg ggcgaaaact ctcaaggatc   4440
   ttaccgctgt tgagatccag ttcgatgtaa cccactcgtg cacccaactg atcttcagca   4500
   tcttttactt tcaccagcgt ttctgggtga gcaaaaacag gaaggcaaaa tgccgcaaaa   4560
   aagggaataa gggcgacacg gaaatgttga atactcatac tcttcctttt tcaatattat   4620
   tgaagcattt atcagggtta ttgtctcatg agcggataca tatttgaatg tatttagaaa   4680
   aataaacaaa taggggttcc gcgcacattt ccccgaaaag tgccacctga cgtctaagaa   4740
   accattatta tcatgacatt aacctataaa aataggcgta tcacgaggcc ctttcgtctt   4800
   cac                                                                 4803